# EUROPEAN PATENT SPECIFICATION

(11) **EP 2 795 325 B1**
(45) Date of publication and mention of the grant of the patent: **13.09.2017**
(21) Application number: 12859548.5
(22) Date of filing: 19.12.2012
(51) Int. Cl.: G01N 33/68

(54) **METHODS FOR DIAGNOSING ALZHEIMER'S DISEASE**
VERFAHREN ZUR DIAGNOSE VON MORBUS ALZHEIMER
MÉTHODES DE DIAGNOSTIC DE LA MALADIE D'ALZHEIMER

(30) Priority: 19.12.2011 US 201161577439 P
(43) Date of publication of application: 29.10.2014
(73) Proprietor: The Washington University, St. Louis, MO 63130 (US)
(72) Inventor: HOLTZMAN, David, St. Louis Missouri 63130 (US); BATEMAN, Randall, St. Louis Missouri 63130 (US); MAWUENYEGA, Kwasi, St. Louis Missouri 63130 (US)
(74) Representative: Turner, Rhiannon Rosalind
(86) International application number: PCT/US2012/070623
(87) International publication number: WO 2013/096451

(56) References cited:
- US-A1- 2011 111 511
- US-A1- 2011 111 511
- US-A1- 2011 143 380
- US-A1- 2011 166 035
- FUKUYAMA R ET AL: "AGE-DEPENDENT CHANGE IN THE LEVELS OF ABETA40 AND ABETA42 IN CEREBROSPINAL FLUID FROM CONTROL SUBJECTS, AND A DECREASE IN THE RATIO OF ABETA 42 TO ABETA40 LEVEL IN CEREBROSPINAL FLUID FROM ALZHEIMER'S DISEASE PATIENTS", EUROPEAN NEUROLOGY, S. KARGER AG, SWITZERLAND, vol. 43, no. 3, 1 January 2000 (2000-01-01), pages 155-160, XP000972956, ISSN: 0014-3022, DOI: 10.1159/000008156
- HANSSON OSKAR ET AL: "Prediction of Alzheimer's disease using the CSF Abeta42/Abeta40 ratio in patients with mild cognitive impairment", DEMENTIA AND GERIATRIC COGNITIVE DISORDERS, KARGER, BASEL, CH, vol. 23, no. 5, 1 January 2007 (2007-01-01), pages 316-320, XP008128561, ISSN: 1420-8008
- GIEDRAITIS ET AL: "The normal equilibrium between CSF and plasma amyloid beta levels is disrupted in Alzheimer's disease", NEUROSCIENCE LETTERS, LIMERICK, IE, vol. 427, no. 3, 29 October 2007 (2007-10-29), pages 127-131, XP022318909, ISSN: 0304-3940, DOI: 10.1016/J.NEULET.2007.09.023
- LEWCZUK P ET AL: "Amyloid [beta] peptides in plasma in early diagnosis of Alzheimer's disease: A multicenter study with multiplexing", EXPERIMENTAL NEUROLOGY, ACADEMIC PRESS, NEW YORK, NY, US, vol. 223, no. 2, 1 June 2010 (2010-06-01), pages 366-370, XP027038377, ISSN: 0014-4886 [retrieved on 2009-08-05]
- K. G. MAWUENYEGA ET AL: "Decreased Clearance of CNS -Amyloid in Alzheimer's Disease", SCIENCE, vol. 330, no. 6012, 9 December 2010 (2010-12-09), pages 1774-1774, XP055240098, US ISSN: 0036-8075, DOI: 10.1126/science.1197623
- BIBL ET AL.: 'CSF amyloid-beta-peptides in Alzheimer's disease, dementia with Lewy bodies and Parkinson's disease dementia'';' BRAIN; vol. 129, May 2006, pages 1177 - 1187, XP055157055
- SHOJI: 'Cerebrospinal fluid Abeta40 and Abeta42: Natural course and clinical usefulness'';' FRONT. BIOSCI. vol. 7, 01 April 2002, pages D997 - D1006, XP055157061

### SEQUENCE LISTING

<110> THE WASHINGTON UNIVERSITY
   HOLTZMAN, David
   BATEMAN, Randall
<120> METHODS FOR DIAGNOSING ALZHEIMER'S DISEASE
<130> 047563-450165
<150> US 61/577,439
   <151> 2011-12-19
<160> 8
<170> PatentIn version 3.5
<210> 1
   <211> 42
   <212> PRT
   <213> Homo sapiens
<400> 1
<210> 2
   <211> 40
   <212> PRT
   <213> Homo sapiens
<400> 2
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val
           35                  40
<210> 3
   <211> 38
   <212> PRT
   <213> Homo sapiens
<400> 3
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly
           35
<210> 4
   <211> 5
   <212> PRT
   <213> Homo sapiens
<400> 4
   Asp Ala Glu Phe Arg
   1               5
<210> 5
   <211> 11
   <212> PRT
   <213> Homo sapiens
<400> 5
   His Asp Ser Gly Tyr Glu Val His His Gln Lys
   1               5                   10
<210> 6
   <211> 12
   <212> PRT
   <213> Homo sapiens
<400> 6
   Leu Val Phe Phe Ala Glu Asp Val Gly Ser Asn Lys
   1 5 10
<210> 7
   <211> 14
   <212> PRT
   <213> Homo sapiens
<400> 7
   Gly Ala Ile Ile Gly Leu Met Val Gly Gly Val Val Ile Ala
   1 5 10
<210> 8
   <211> 12
   <212> PRT
   <213> Homo sapiens
<400> 8
   Gly Ala Ile Ile Gly Leu Met Val Gly Gly Val Val
   1 5 10

## Description

### GOVERNMENTAL RIGHTS

This invention was made with government support under R-01-NS065667 awarded by The National Institutes of Health. The government has certain rights in the invention.

### FIELD OF THE INVENTION

The invention relates to methods for the diagnosis of Aβ amyloidosis.

### BACKGROUND OF INVENTION

Alzheimer's Disease (AD) is the most common cause of dementia and is an increasing public health problem. It is currently estimated to afflict 5 million people in the United States, with an expected increase to 13 million by the year 2050 (Herbert et al 2001, Alzheimer Dis. Assoc. Disord. 15(4): 169-173). AD, like other central nervous system (CNS) degenerative diseases, is characterized by disturbances in protein production, accumulation, and clearance. In AD, dysregulation in the metabolism of the protein, amyloid-beta (Aβ), is indicated by a massive buildup, or Aβ amyloidosis, of this protein in the brains of those with the disease. AD leads to loss of memory, cognitive function, and ultimately independence. It takes a heavy personal and financial toll on the patient and the family. Because of the severity and increasing prevalence of this disease in the population, it is urgent that better treatments be developed.

Currently, there are some medications that modify symptoms, however, there are no disease-modifying treatments. Disease-modifying treatments will likely be most effective when given *before* the onset of permanent brain damage. However, by the time clinical diagnosis of AD is made, extensive neuronal loss has already occurred (Price et al. 2001, Arch. Neurol. 58(9): 1395-1402). Currently, there are no means of diagnosing individuals at risk of developing AD before the onset of clinical symptoms or of effectively measuring the effects of treatments that may prevent the onset or slow the progression of the disease.

A need exists, therefore, for a way to identify those at risk of developing AD. Early identification and the ability to measure the effects of drugs that target Aβ generation would be most helpful in preventing or delaying the onset of AD, and enhancing drug development efforts.

### BRIEF DESCRIPTION OF THE DRAWINGS

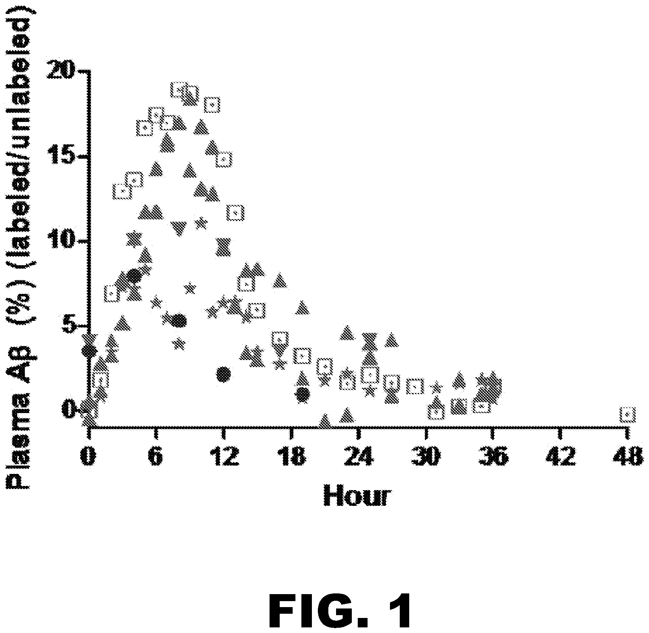

**FIG. 1** depicts a graph showing the percent labeled Aβ in blood over 48 hours for 6 participants. Note the rapid rise to plateau by 9 hours with a rapid clearance rate.

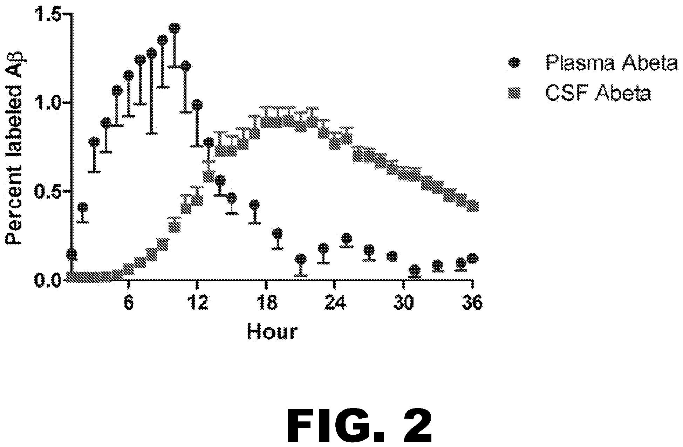

**FIG. 2** depicts a graph showing the percent labeled Aβ in blood over 36 hours for 6 participants (blue circles) and percent labeled Aβ in CSF in twelve participants (red squares).

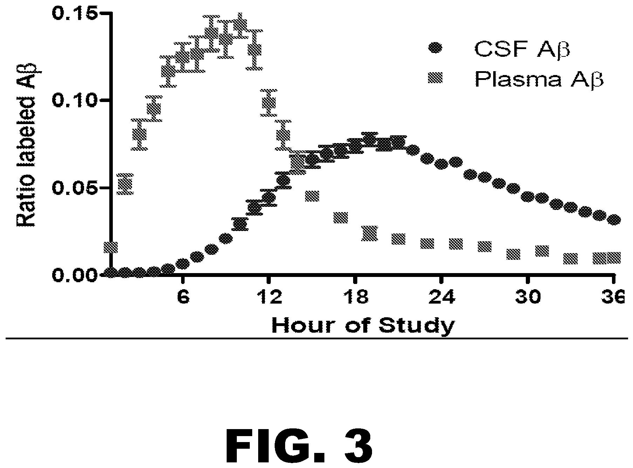

**FIG. 3** depicts a graph showing that blood Aβ kinetics are faster than CNS Aβ labeling kinetics. Blood and CSF samples were collected from AD participants during and after intravenous labeling for 9 hours. Plasma Aβ labeling increases within 1 hour, while CSF Aβ labeling does not increase until 5 hours. In older participants, the half-life of blood Aβ is 2.1 hours, while the half-life of CNS Aβ is 11.6 hours.

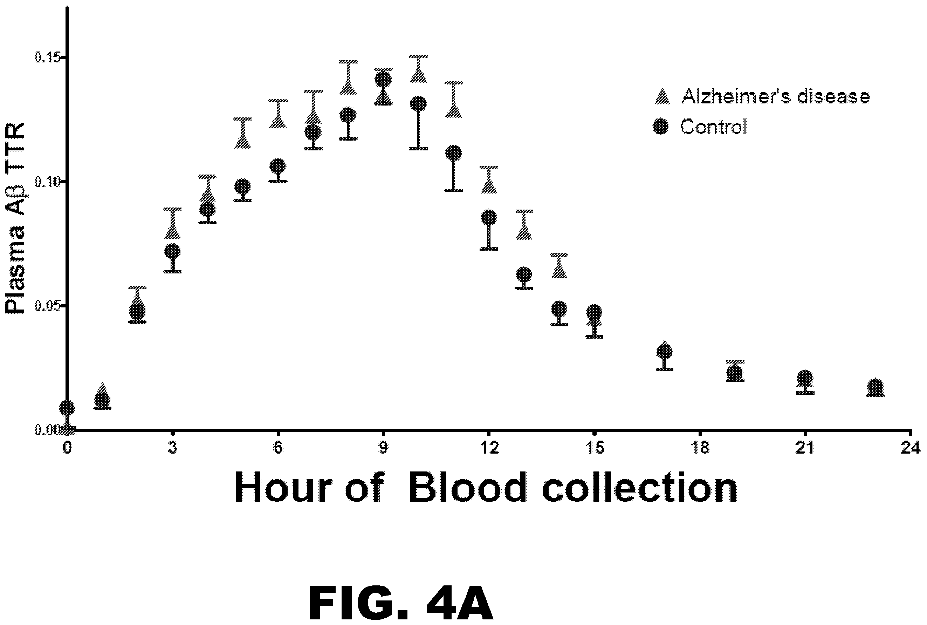

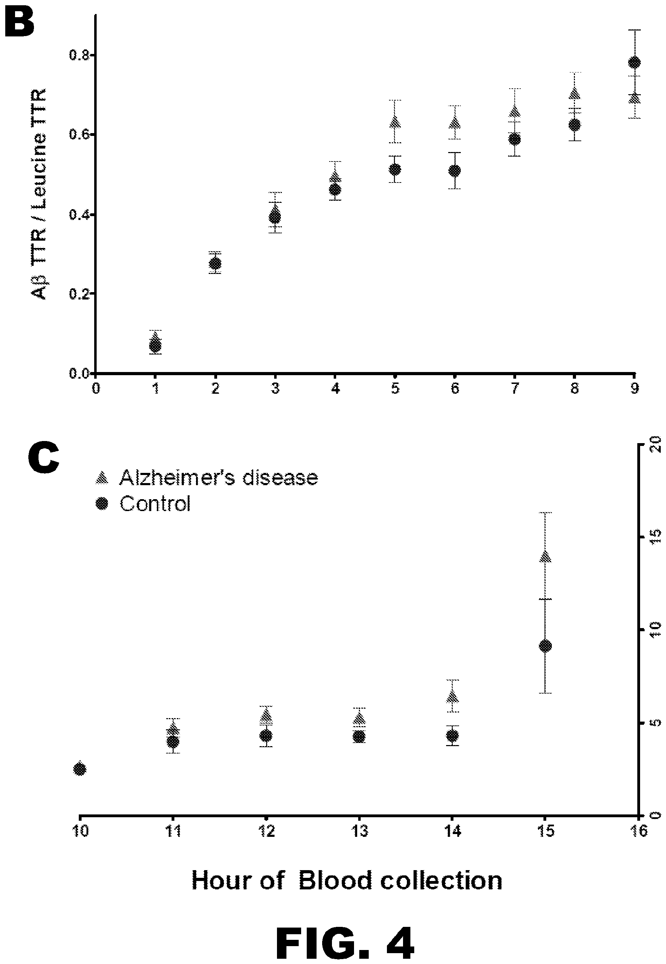

**FIG. 4** depicts blood Aβ kinetics during and after intravenous labeling. **A.** Blood samples from AD participants and controls during and after 9 hours of intravenous labeling. During and after the labeling phase, the AD group has increased labeled Aβ over the first 15 hours. **B** and **C..** Normalized labeled plasma Aβ. Labeled plasma Aβ was normalized by labeled plasma leucine for each hour. (Hours 0-9 are depicted in **4B** while hours 10-15 are depicted in **4C**.) Increased normalized labeled plasma Aβ is noted from hours 5 to 15 in AD compared to control.

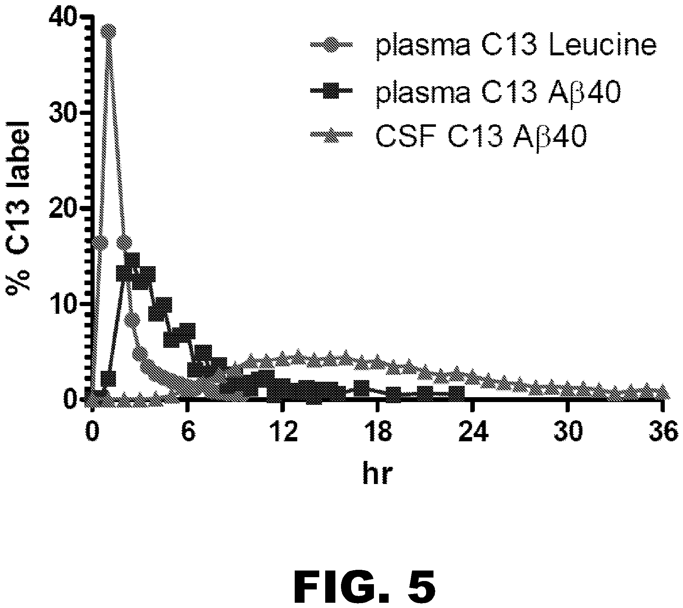

**FIG. 5** depicts Aβ kinetics in blood after oral labeling. A participant was given a single oral dose of labeled leucine at time zero. Blood samples were collected every hour for 48 hours. Plasma labeled Aβ was measured and the kinetics curve is shown. A subsequent ascending dose oral labeling study revealed that plasma Aβ can be sufficiently labeled with small doses of oral labeled leucine. The labeled CSF Aβ curve is shown for comparison. Note the significantly faster half-life of blood Aβ and the extended time course captured to measure later secondary clearance effects.

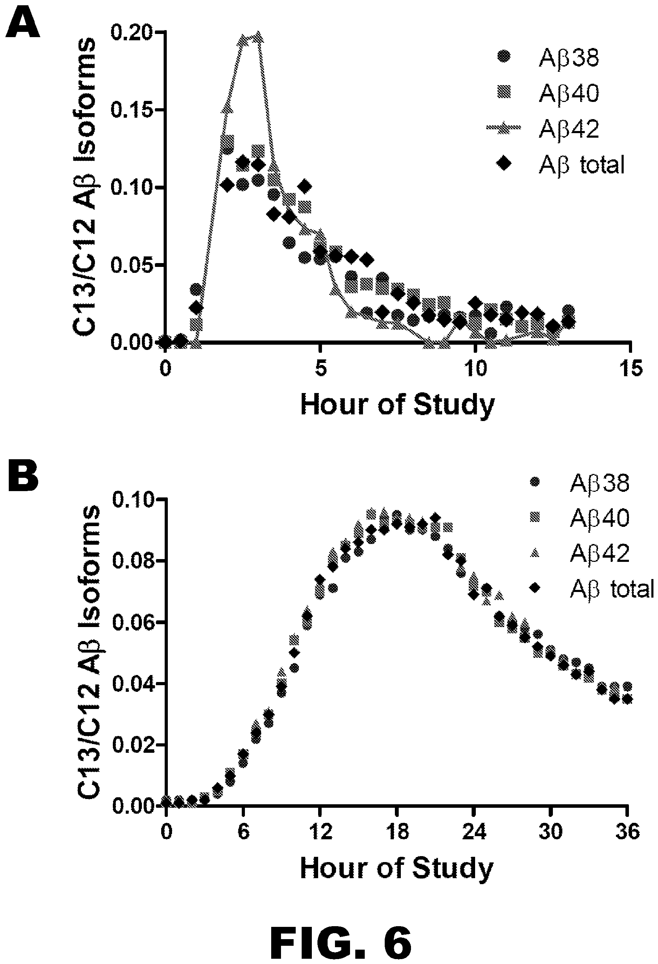

**FIG. 6** depicts a graph showing Aβ total, 42, 40, and 38 variant quantitation over time. Because specific Aβ variant kinetics (e.g. Aβ42) may be altered in AD, a novel method was developed to allow for the sensitive and specific measurement of labeled Aβ variants. Aβ variants were immunoprecipitated from pulse oral labeled plasma samples and quantified for kinetics **(A).** Note that labeled Aβ42 had an increased peak and faster clearance (half-life ∼1 hour) compared to other Aβ variants (in this cognitively normal participant). Aβ variants were similarly processed from steady-state IV labeled CSF samples and quantified for kinetics (**B**). Note the similar metabolism in the CSF of this cognitively normal, PET PiB negative participant.

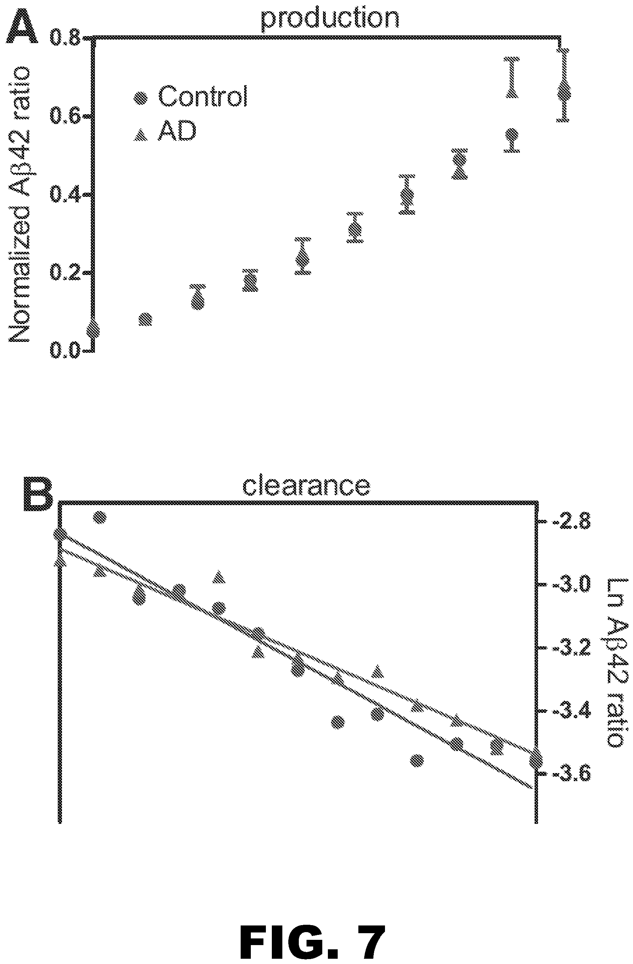

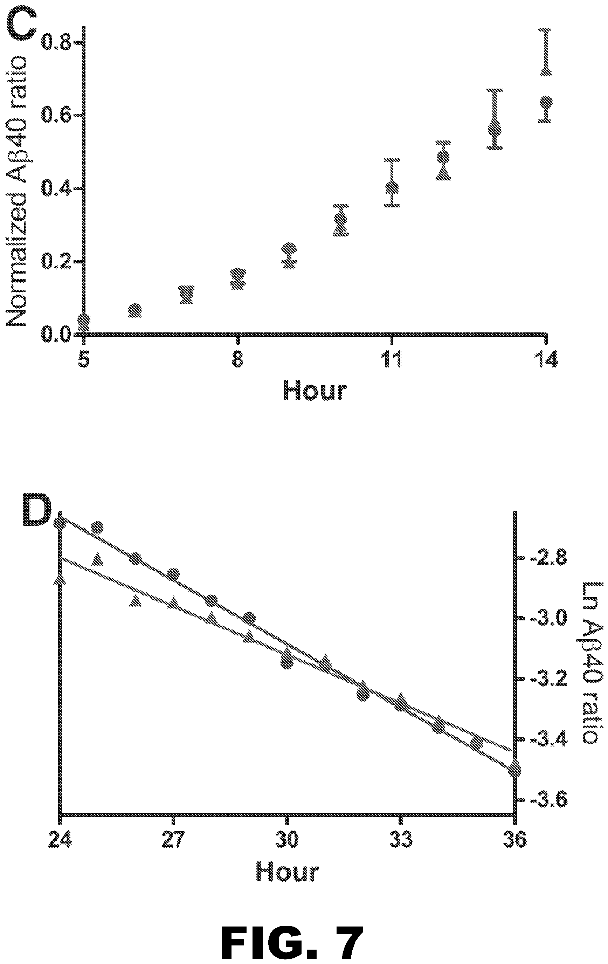

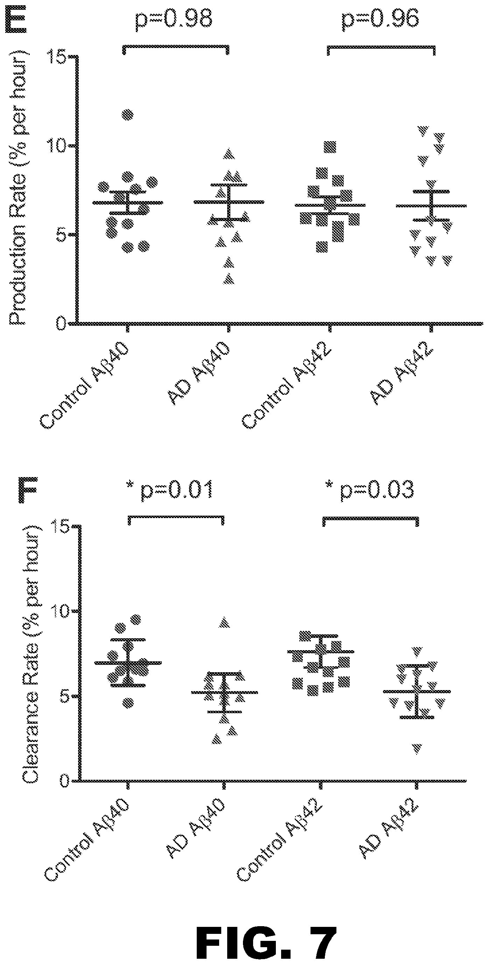

**FIG. 7** depicts Aβ kinetics in the CNS of 12 AD participants (red triangles) and 12 controls (blue circles). The amount of labeled Aβ42 and Aβ40 was measured and compared between groups to measure production and clearance rates of both Aβ species. Error bars indicate SEM. **(A)** Normalized labeled Aβ42 production phase. (**B**) Aβ42 clearance phase. (**C**) Normalized labeled Aβ40 production phase. (**D**) Aβ40 clearance phase. (**E**) Fractional synthesis rates of Aβ42 and Aβ40. (**F**) Fractional clearance rates of Aβ42 and Aβ40.

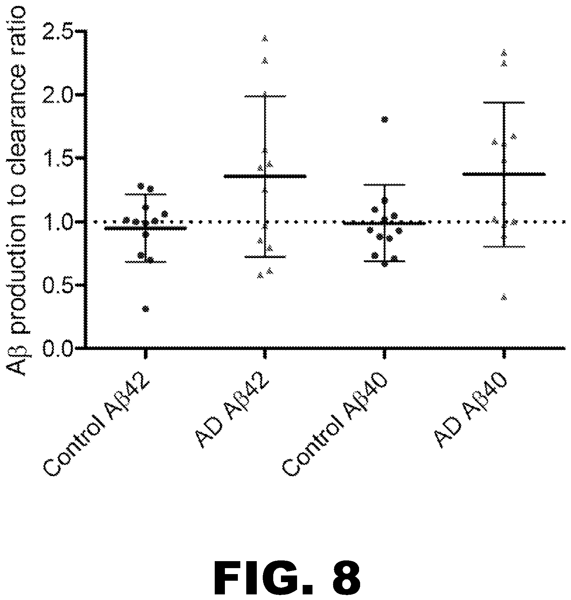

**FIG. 8** graphically depicts the ratio of Aβ production to clearance in AD and controls. The ratio of Aβ42 production to clearance rate is balanced in controls (0.95), while it is higher in those with AD (1.35). Similarly, there is increased Aβ40 production to clearance ratios in AD (1.37) compared to controls (0.99). Means ± standard deviations are plotted (p<0.05 ANOVA).

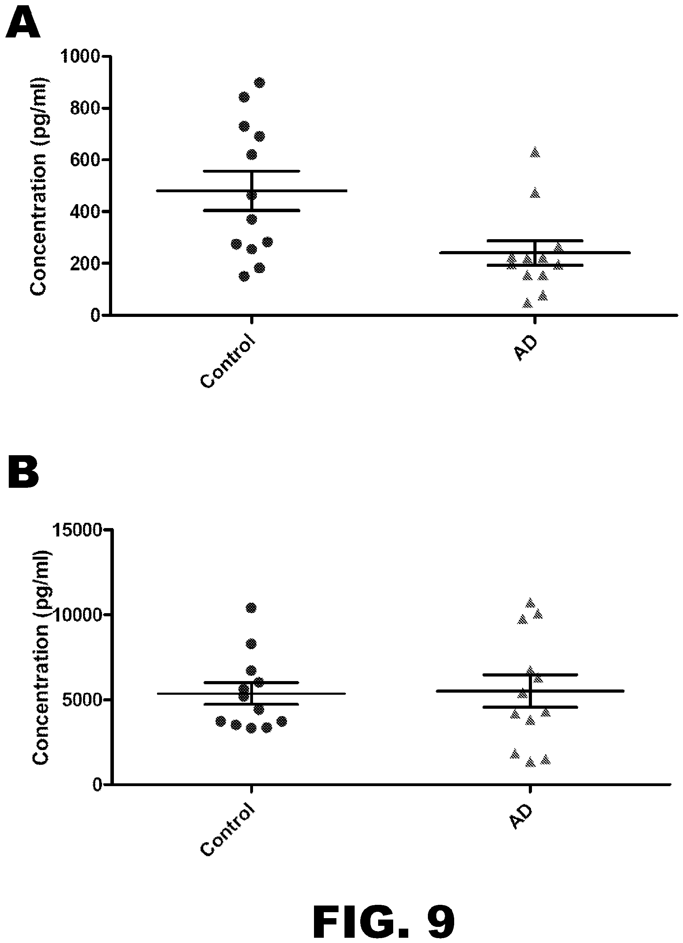

**FIG. 9** graphically depicts average CSF **(A)** and Aβ40 **(B)** concentrations. As expected CSF Aβ42 concentrations were lower in the AD group compared to the control group (p<0.05). Means ± standard errors are plotted.

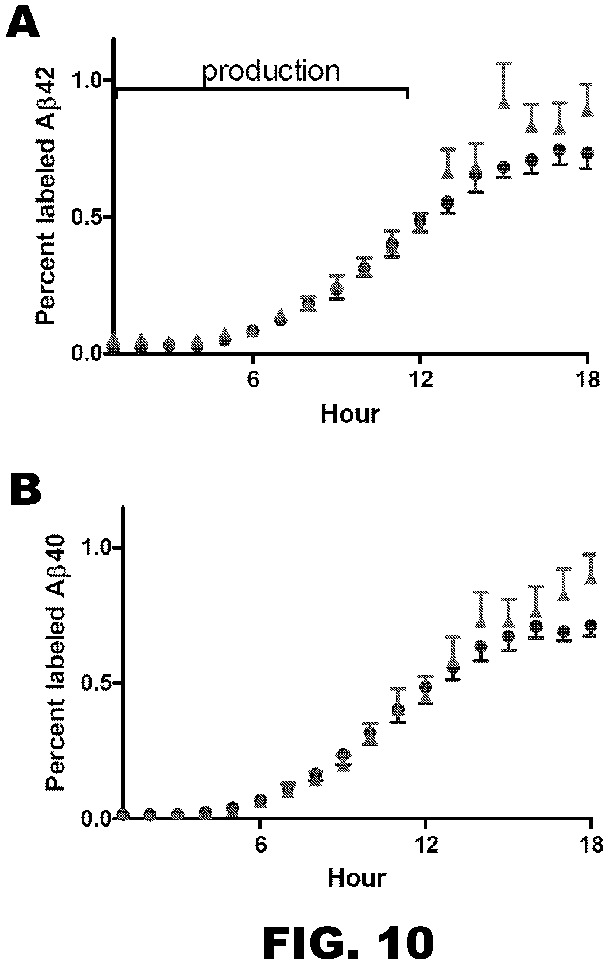

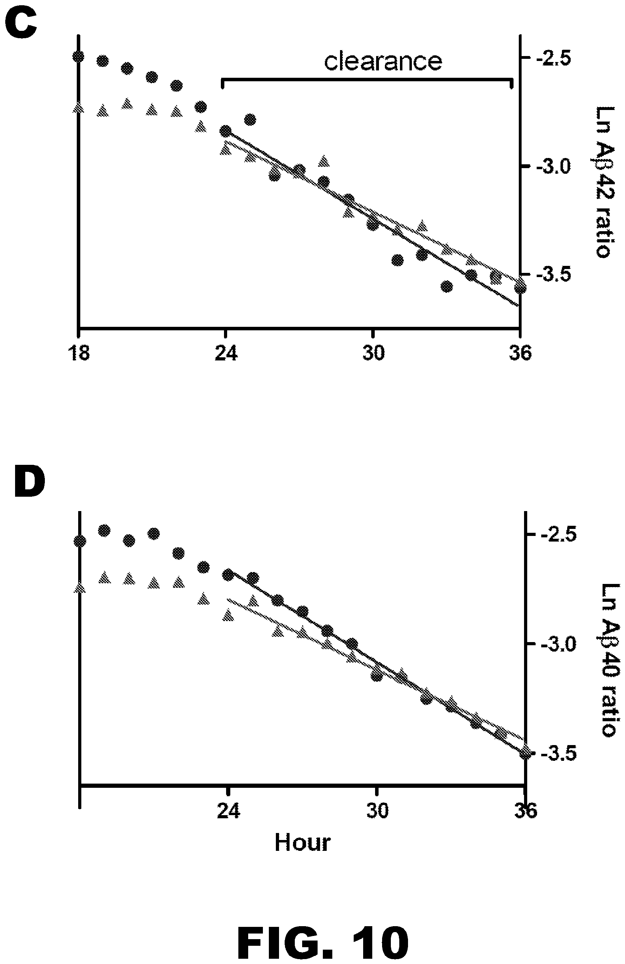

**FIG. 10** graphically depicts complete time courses of the average Aβ kinetics in the CNS of twelve AD participants (red triangles) and twelve controls (blue circles). The average leucine normalized labeled Aβ42 **(A)** and Aβ40 **(B)** time course is shown for hours 1 to 18. The natural log (ln) plot of labeled to unlabeled ratio of Aβ42 (**C**) and Aβ40 (**D**) is shown for hours 18 to 36.

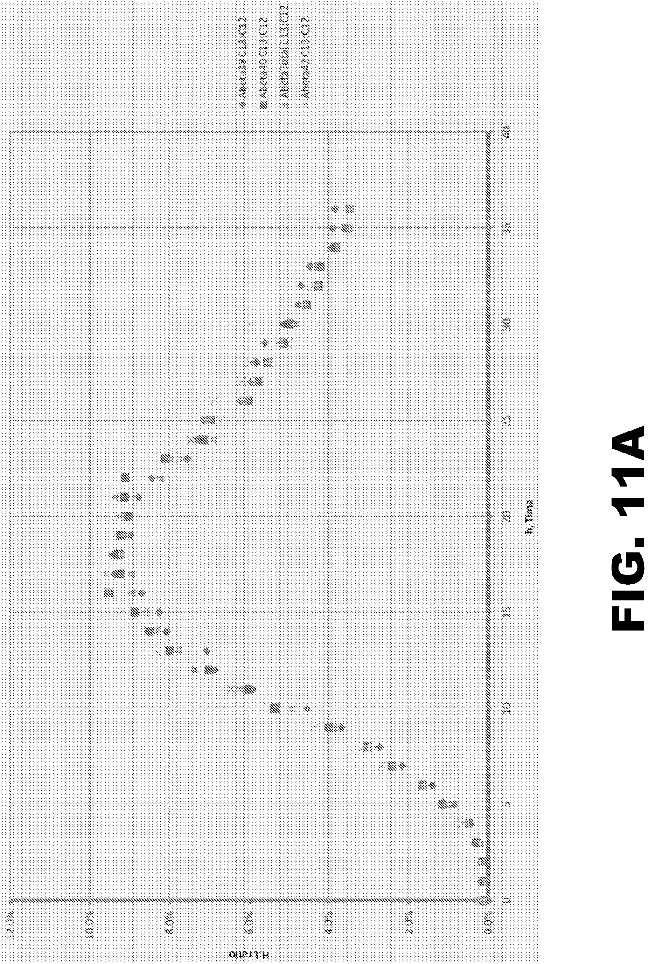

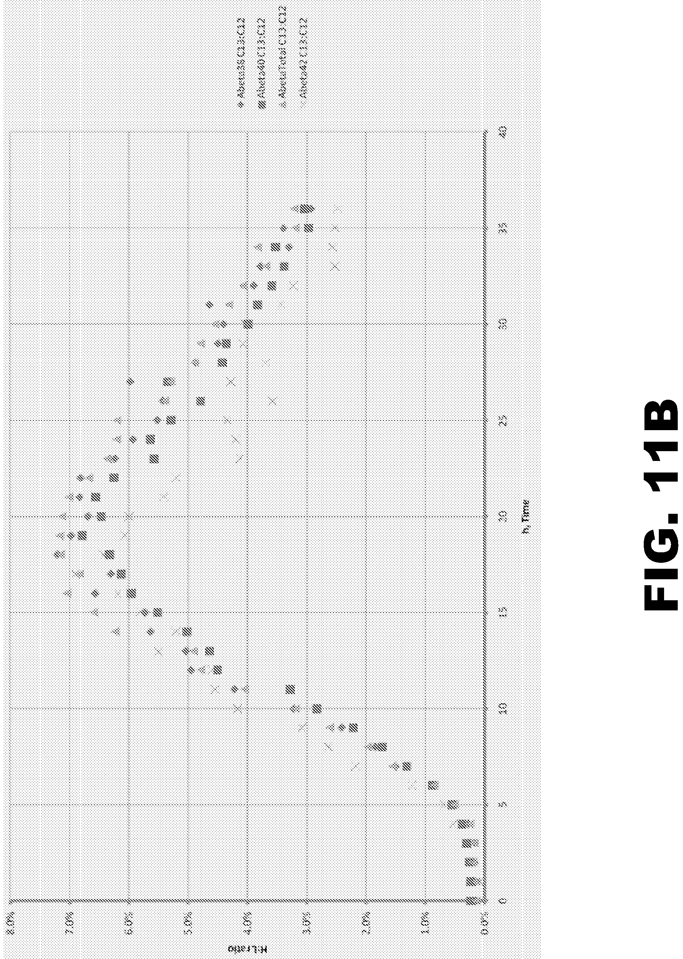

**FIG. 11** depicts two graphs plotting the H:L ratio of ¹³C₆-Leu/¹²C₆-Leu over time. **(A)** Samples from a normal person with no amyloid deposition by PIB, and **(B)** samples from an AD individual with positive amyloid deposition.

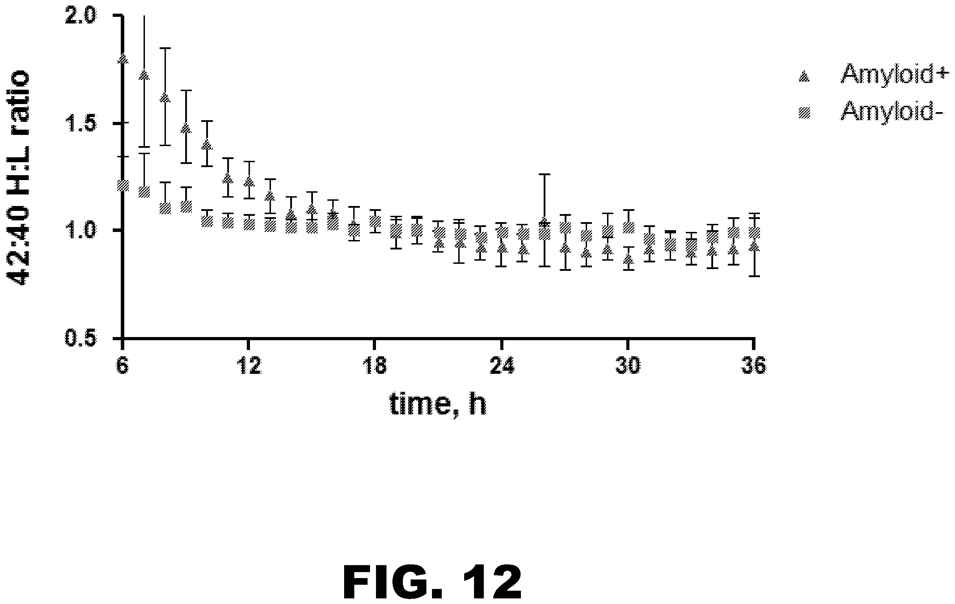

**FIG. 12** depicts a graph plotting the Aβ42:Aβ40 H:L ratio of ¹³C₆-Leu/¹²C₆-Leu over time in 50 AD patients.

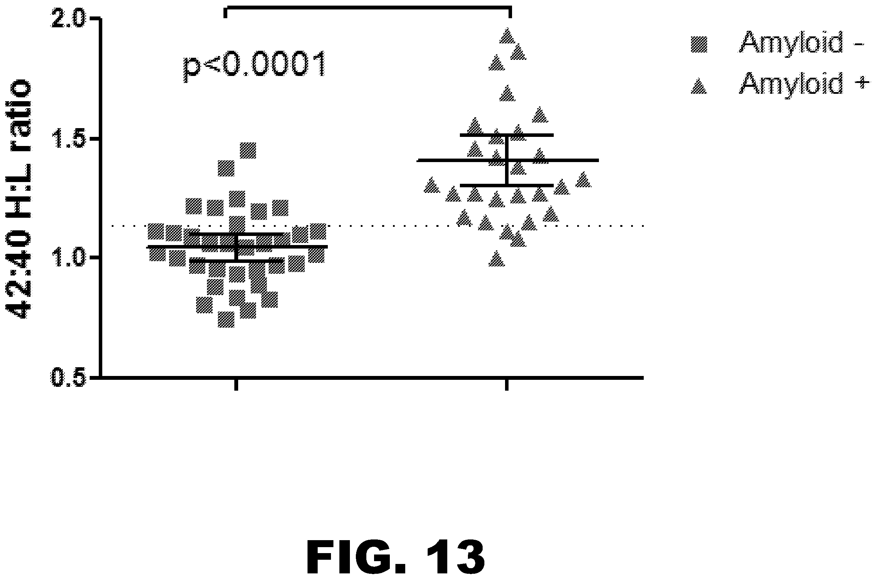

**FIG. 13** depicts a plot showing the relative labeling of Aβ42:Aβ40 H:L ratio of ¹³C₆-Leu/¹²C₆-Leu at 10 hrs.

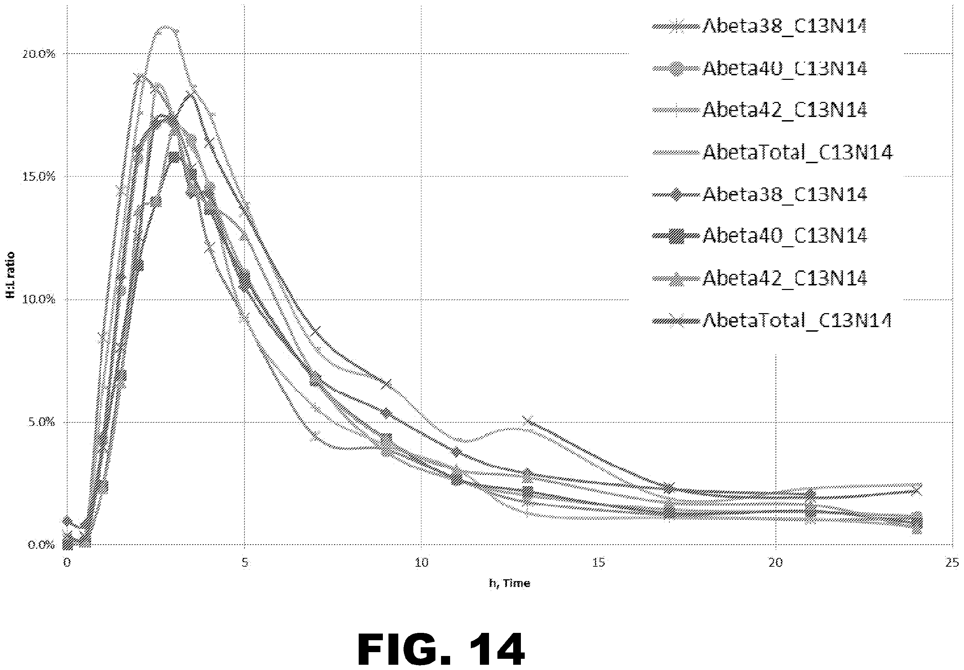

**FIG. 14** depicts a plot showing the relative labeling of different Aβ isoforms H:L ratio of ¹³C₆-Leu/¹²C₆-Leu over time.

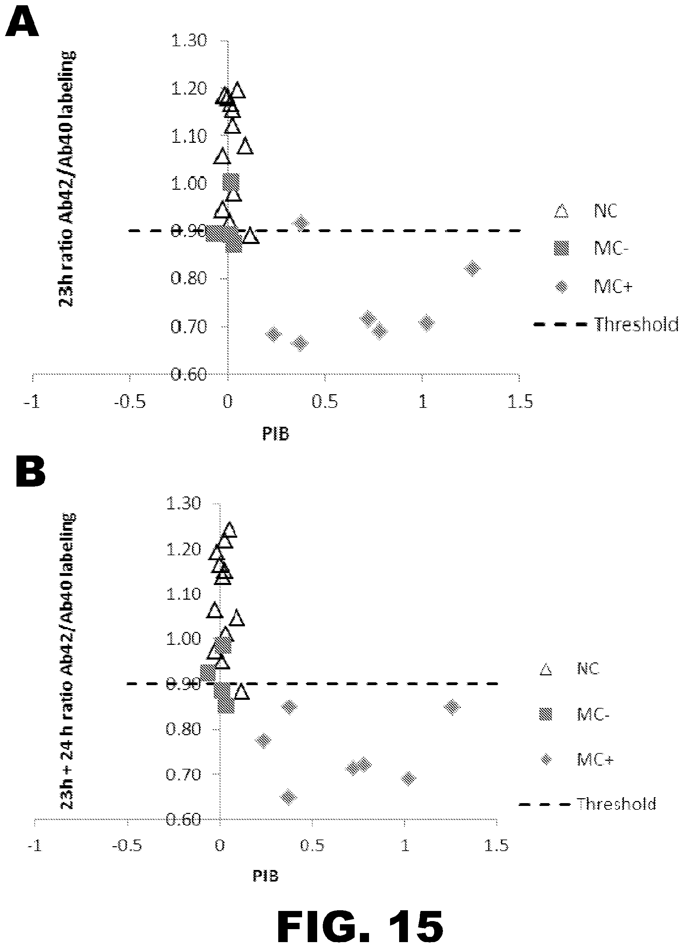

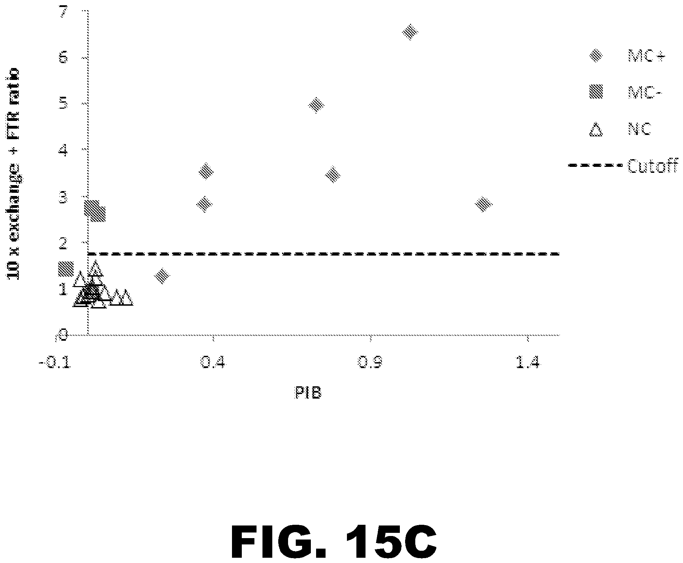

**FIG. 15** depicts three graphs showing that a comparison of isotopic enrichments around the midpoint on the back end of the kinetic tracer curve is able to discriminate the PIB groups highly significantly. **FIG. 15A** shows the ratio of Aβ42 percent labeled / Aβ40 percent labeled at 23 hours graphed on the y-axis and PIB staining graphed on the x-axis. A threshold ratio of 0.9 is indicated by the dashed line. **FIG. 15B** shows the average of the ratio of Aβ42 percent labeled / Aβ40 percent labeled at 23 hours and 24 hours graphed on the y-axis and PIB staining graphed on the x-axis. A threshold ratio of 0.9 is indicated by the dashed line. **FIG. 15C** shows the calculated values of ten times kₑₓ₄₂ added to ratio of the rate constants for irreversible loss for Aβ42 versus Aβ40 (10x kₑₓ₄₂ + FTR ratio) plotted as a function of PIB staining. A threshold ratio of 1.75 is indicated by the dashed line. MC+ = patients with PSEN1 or PSEN2 mutations that were PIB positive by PET; MC- = patients with PSEN1 or PSEN2 mutations that were PIB negative by PET; NC = non-carrier mutation carrier sibling controls.

### DETAILED DESCRIPTION OF THE PREFERRED EMBODIMENTS

A method of diagnosing of Aβ amyloidosis in a subject has been developed. The invention greatly enhances the accuracy of detection of Aβ amyloidosis early in the course of clinical disease or prior to the onset of brain damage and clinical symptoms in patients at risk of developing AD and monitoring the progression of the disease. Advantageously, as illustrated in the examples, the method allows for measuring Aβ dynamics in the blood without invasive spinal catheters. In addition, the method allows for specific testing of proposed disease modifying therapeutics which target Aβ.

### I. Methods for monitoring the in vivo metabolism of neurally derived biomolecules

The current invention provides methods for diagnosing Aβ amyloidosis resulting from differential kinetics of amyloid-β (Aβ) protein and Aβ protein variants in a subject. Alzheimer's Disease (AD) is a debilitating disease characterized by accumulation of amyloid plaques (Aβ amyloidosis) in the central nervous system (CNS) resulting from increased production, decreased clearance, or both, of amyloid-β (Aβ) protein and Aβ protein variants. Non-limiting examples of symptoms associated with Aβ amyloidosis may include impaired cognitive function, altered behavior, abnormal language function, emotional dysregulation, seizures, impaired nervous system structure or function, and an increased risk of development of Alzheimer's disease or cerebral amyloid angiopathy.

In some embodiments, comparing the level of a Aβ variant in a subject to the level of the Aβ variant in an individual with no amyloidosis, one skilled in the art may be able to diagnose Aβ amyloidosis in a subject before the development of symptoms associated with Aβ amyloidosis. In other embodiments, by comparing the level of an Aβ variant in a subject to the level of another Aβ variant in the subject, one skilled in the art may be able to diagnose Aβ amyloidosis in the subject before the development of symptoms associated with Aβ amyloidosis. In addition, the invention permits the measurement of the pharmacodynamic effects of disease-modifying therapeutics in a subject.

### (a) Aβ variants

The method of the invention comprises measuring the *in vivo* levels of one or more Aβ variants. As used herein, the term "Aβ variant" refers to total Aβ protein, Aβ40, Aβ42, Aβ38, or another Aβ isoform. In some embodiments, the *in vivo* levels of Aβ variants may be measured by measuring the *in vivo* levels of labeled Aβ variants. Non-limiting examples of Aβ variants whose *in vivo* levels may be measured may include total Aβ protein, Aβ40, Aβ42, or Aβ38. In one embodiment, the *in vivo* levels of labeled Aβ protein may be measured. In another embodiment, the *in vivo* levels of labeled Aβ40 may be measured. In yet another embodiment, the *in vivo* levels of labeled Aβ42 may be measured. In another embodiment, the *in vivo* levels of labeled Aβ38 may be measured.

In other embodiments, the *in vivo* levels of Aβ variants may be measured by measuring the *in vivo* relative labeling of Aβ variants. As used herein, "in vivo relative labeling" refers to the percent of the variant that is labeled in vivo. Non-limiting examples of Aβ variants whose *in vivo* relative labeling may be measured may include Aβ protein, Aβ40, Aβ42, or Aβ38. In one embodiment, the *in vivo* relative labeling of Aβ protein may be measured. In another embodiment, the *in vivo* relative labeling of Aβ40 may be measured. In yet another embodiment, the *in vivo* relative labeling of Aβ42 may be measured. In another embodiment, the *in vivo* relative labeling of Aβ38 may be measured.

In some embodiments, the *in vivo* relative labeling of more than one Aβ protein or Aβ protein variant may be measured *in vivo* in the subject. In some embodiments, the *in vivo* relative labeling of Aβ protein and Aβ42 may be measured. In other embodiments, the *in vivo* relative labeling of Aβ protein and Aβ40 may be measured. In yet other embodiments, the *in vivo* relative labeling of Aβ protein and Aβ38 may be measured. In additional embodiments, the *in vivo* relative labeling of Aβ40 protein and Aβ38 may be measured. In still other embodiments, the *in vivo* relative labeling of Aβ protein and Aβ38 may be measured. In other embodiments, the *in vivo* relative labeling of Aβ protein, Aβ42 and Aβ40 may be measured. In yet other embodiments, the *in vivo* relative labeling of Aβ protein, Aβ40 and Aβ38 may be measured. In still other embodiments, the *in vivo* relative labeling of Aβ protein, Aβ42 and Aβ38 may be measured. In additional embodiments, the *in vivo* relative labeling of Aβ42, Aβ40 and Aβ38 may be measured. In preferred embodiments, the *in vivo* relative labeling of Aβ42 protein and Aβ40 may be measured.

Those of skill in the art will recognize that measuring the *in vitro* digestion products of Aβ (e.g., Aβ₆₋₁₆, Aβ₁₇₋₂₈) may be used to determine the *in vivo* metabolism of the Aβ protein and Aβ variants comprising the Aβ *in vitro* digestion products. In some embodiments, the *in vivo* relative labeling of one or more Aβ variants may be measured by measuring *in vitro* digestion products of Aβ (e.g., Aβ₆₋₁₆, Aβ₁₇₋₂₈).

### (b) measuring in vivo relative labeling

The *in vivo* relative labeling of Aβ variants may be measured by labeling an Aβ variant as it is synthesized in the central nervous system *in vivo,* and measuring the labeling of the Aβ variant over time. These measurements may be used to calculate the ratio or percent labeled Aβ variant.

### i. labeling moiety

An Aβ variant may be labeled *in vivo* as it is synthesized in the central nervous system using a labeled moiety. Several different moieties may be used to label the Aβ variant. Generally speaking, the two types of labeling moieties typically utilized in the method of the invention are radioactive isotopes and non-radioactive (stable) isotopes. In a preferred embodiment, non-radioactive isotopes may be used and measured by mass spectrometry. Preferred stable isotopes include deuterium ²H, ¹³C, ¹⁵N, ^{17 or 18}O, ^{33,34, or 36}S, but it is recognized that a number of other stable isotope that change the mass of an atom by more or less neutrons than is seen in the prevalent native form would also be effective. A suitable label generally will change the mass of the biomolecule under study such that it can be detected in a mass spectrometer. In one embodiment, the labeled moiety is an amino acid comprising a non-radioactive isotope (e.g., ¹³C). Alternatively, a radioactive isotope may be used, and the labeled biomolecules may be measured with a scintillation counter rather than a mass spectrometer. One or more labeled moieties may be used simultaneously or in sequence.

Those of skill in the art will appreciate that several amino acids may be used to provide the label of Aβ variant. Generally, the choice of amino acid is based on a variety of factors such as: (1) The amino acid generally is present in at least one residue of the Aβ variant. (2) The amino acid is generally able to quickly reach the site of protein synthesis and rapidly equilibrate across the blood-brain barrier. Leucine is a preferred amino acid to label proteins that are synthesized in the CNS such as Aβ variants. (3) The amino acid ideally may be an essential amino acid (not produced by the body), so that a higher percent of labeling may be achieved. Non-essential amino acids may also be used; however, measurements will likely be less accurate. (4) The amino acid label generally does not influence the metabolism of the protein of interest (e.g., very large doses of leucine may affect muscle metabolism). And (5) availability of the desired amino acid (i.e., some amino acids are much more expensive or harder to manufacture than others). In one embodiment, ¹³C₆-phenylalanine, which contains six ¹³C atoms, is used to label an Aβ variant. In a preferred embodiment, ¹³C₆-leucine is used to label an Aβ variant.

There are numerous commercial sources of labeled amino acids, both non-radioactive isotopes and radioactive isotopes. Generally, the labeled amino acids may be produced either biologically or synthetically. Biologically produced amino acids may be obtained from an organism (e.g., kelp/seaweed) grown in an enriched mixture of ¹³C, ¹⁵N, or another isotope that is incorporated into amino acids as the organism produces proteins. The amino acids are then separated and purified. Alternatively, amino acids may be made with known synthetic chemical processes.

### ii. administration of the labeled moiety

The method of the invention provides that the labeled moiety may be administered to the subject. The labeled moiety may be administered to a subject by several methods. Suitable methods of administration include intravenously, intra-arterially, subcutaneously, intraperitoneally, intramuscularly, or orally. In a preferred embodiment, the labeled moiety is administered by intravenous infusion. In another preferred embodiment, the labeled moiety may be orally ingested.

The labeled moiety may be administered slowly over a period of time or as a large single dose depending upon the type of analysis chosen (e.g., steady state or bolus/chase). To achieve steady-state levels of the labeled Aβ variant, the labeling time generally should be of sufficient duration so that the labeled Aβ variant may be reliably quantified. In one embodiment, the labeled moiety is labeled leucine and the labeled leucine is administered intravenously for nine hours. In another embodiment, the labeled leucine is administered intravenously for 12 hours. In yet another embodiment, the labeled leucine is administered orally. In another embodiment, the labeled leucine is administered orally as a single bolus for pulse labeling.

Those of skill in the art will appreciate that the amount (or dose) of the labeled moiety can and will vary. Generally, the amount is dependent on (and estimated by) the following factors. (1) The type of analysis desired. For example, to achieve a steady state of about 15% labeled leucine in plasma requires about 2 mg/kg/hr over 9 hr after an initial bolus of 2 mg/kg over 10 min. In contrast, if no steady state is required, a large bolus of labeled leucine (e.g., 1 or 5 grams of labeled leucine) may be given. (2) The Aβ variant under analysis. For example, if the Aβ variant is being produced rapidly, then less labeling time may be needed and less label may be needed - perhaps as little as 0.5 mg/kg over 1 hour. However, most Aβ variants have half-lives of hours to days and, so more likely, a continuous infusion for 4, 9 or 12 hours may be used at 0.5 mg/kg to 4 mg/kg. And (3) the sensitivity of detection of the label. For example, as the sensitivity of label detection increases, the amount of label that is needed may decrease.

Those of skill in the art will appreciate that more than one label may be used in a single subject. This would allow multiple labeling of the same Aβ variant and may provide information on the production or clearance of that Aβ variant at different times. For example, a first label may be given to a subject over an initial time period, followed by a pharmacologic agent (drug), and then a second label may be administered. In general, analysis of the samples obtained from this subject would provide a measurement of metabolism before AND after drug administration, directly measuring the pharmacodynamic effect of the drug in the same subject.

Alternatively, multiple labels may be used at the same time to increase labeling of the Aβ variant, as well as obtain labeling of a broader range of Aβ variants.

### iii. biological sample

The method of the invention provides that a biological sample be obtained from a subject so that the *in vivo* metabolism of the labeled Aβ variant may be determined. Suitable biological samples include cerebral spinal fluid (CSF), blood plasma and blood serum. In one embodiment of the invention, biological samples are taken from the CSF. In a preferred embodiment, biological samples are collected from the blood. As used herein, "blood" refers to either blood plasma or blood serum.

Cerebrospinal fluid may be obtained by lumbar puncture with or without an indwelling CSF catheter (a catheter is preferred if multiple collections are made over time). Blood may be collected by veni-puncture with or without an intravenous catheter, and processed according to methods known in the art. Urine may be collected by simple urine collection or more accurately with a catheter. Saliva and tears may be collected by direct collection using standard good manufacturing practice (GMP) methods.

After administration of a labeled amino acid, one or more samples will be collected from the subject. As will be appreciated by those of skill in the art, the number of samples and when they would be taken generally will depend upon a number of factors such as: the type of analysis, type of administration, the Aβ variant of interest, the rate of metabolism, the type of detection, etc. In some embodiments, the invention provides that a first biological sample be taken from the subject prior to administration of the label to provide a baseline for the subject.

In some embodiments, samples of blood or CSF are taken hourly for 36 hours. Alternatively, samples may be taken every other hour or even less frequently. In one embodiment, samples of CSF are taken about 5, 6, 7, 8, 9, 10, 11, 12, 13, 14, or 15 hours after administration of the labeled moiety. In another embodiment, samples of CSF are taken about 9, 10, or 11 hours after administration of the labeled moiety. In yet another embodiment, samples of CSF are taken about 20, 21, 22, 23, 24, 25, 26, 27, 28, 29, 30, 31, 32, 33, 34, 35, or 36 hours after administration of the labeled moiety. In yet another embodiment, samples of CSF are taken about 26, 27, or 28 hours after administration of the labeled moiety. In a preferred embodiment, samples of CSF are taken about 10 hours after administration of the labeled moiety. In another preferred embodiment, samples of CSF are taken about 27 hours after administration of the labeled moiety.

In one embodiment, blood samples are taken about 1, 2, 3, 4, 5, 6, 7, 8, or 9 hours after administration of the labeled moiety. In another embodiment, blood samples are taken about 5, 6, 7, 8, 9, 10, 11, or 12 hours after administration of the labeled moiety. In yet another embodiment, blood samples are taken about 17, 18, 19, 20, 21, 22, 23, 24, 25, or 26 hours after administration of the labeled moiety

In general, biological samples obtained during the production phase may be used to determine the rate of synthesis of the Aβ variant, and biological samples taken during the clearance phase may be used to determine the clearance rate of the Aβ variant. For CSF, the production phase is typically in the first 12 hours after labeling, while the clearance phase is typically 24 to 36 hours after labeling. For blood, the production phase is typically the first three hours after labeling, while the clearance phase is typically between about 4 to about 10 hours after labeling. In an alternative embodiment, samples may be taken from an hour to days or even weeks apart depending upon the protein's synthesis and clearance rate.

### iv. detection

The present invention provides that detection of the amount of labeled Aβ and the amount of unlabeled Aβ in the biological samples may be detected. Suitable methods for the detection of labeled and unlabeled Aβ can and will vary according to the type of labeled moiety used to label it. If the labeled moiety is a non-radioactively labeled amino acid, then the method of detection typically should be sensitive enough to detect changes in mass of the labeled protein with respect to the unlabeled protein. In a preferred embodiment, mass spectrometry is used to detect differences in mass between the labeled and unlabeled Aβ. In one embodiment, gas chromatography mass spectrometry is used. In an alternate embodiment, MALDI-TOF mass spectrometry is used. In a preferred embodiment, high-resolution tandem mass spectrometry is used.

Additional techniques may be utilized to separate Aβ from other proteins and biomolecules in the biological sample. As an example, immunoprecipitation may be used to isolate and purify Aβ before it is analyzed by mass spectrometry. Alternatively, mass spectrometers having chromatography setups may be used to isolate proteins without immunoprecipitation, and then Aβ may be measured directly. In an exemplary embodiment, Aβ is immunoprecipitated and then analyzed by a liquid chromatography system interfaced with a tandem MS unit equipped with an electrospray ionization source (LC-ESI-tandem MS).

The invention also provides that multiple Aβ variants in the same biological sample may be measured simultaneously. That is, both the amount of unlabeled and labeled Aβ may be detected and measured separately or at the same time for multiple Aβ variants. As such, the invention provides a useful method for screening changes in synthesis and clearance of Aβ variants on a large scale (i.e. proteomics/metabolomics) and provides a sensitive means to detect and measure Aβ variants involved in the underlying pathophysiology of AD.

### v. ratio of relative labeling of Aβ variants

The amount of labeled and unlabeled Aβ variant in a biological sample may be used to determine the relative labeling of the Aβ variant in the sample. According to the invention, the relative labeling of an Aβ variant in a sample is the ratio of labeled Aβ variant to unlabeled Aβ variant in the sample. Those of skill in the art will recognize that the relative labeling of an Aβ variant in a biological sample is directly proportional to the metabolism of said Aβ variant in the subject.

### (c) ratio of relative labeling and diagnosis

A method of the invention may comprise calculating the ratio of relative labeling of one Aβ variant relative to the relative labeling of another Aβ variant. Those of skill in the art will recognize that, when the relative labeling of any two Aβ variants are similar in a given sample, the ratio of relative labeling of said two Aβ variants may be about 1. Conversely, when the relative labeling of any one Aβ variant differs from the relative labeling of other Aβ variants in a given sample, the ratio of relative labeling of said Aβ variant to another Aβ variant in the biological sample may be a number other than 1 (i.e. greater than 1 or less than 1).

As illustrated in the examples, the inventors discovered that the relative labeling of Aβ variants in healthy subjects are similar in a given sample taken at a given time. Therefore, the ratio of relative labeling of any Aβ variant to any other Aβ variant in a healthy subject may be about 1. Surprisingly, the inventors also discovered that the relative labeling of Aβ42 in subjects with Aβ amyloidosis is different from the relative labeling of other Aβ variants in a given sample taken at a given time. Therefore, the ratio of relative labeling of Aβ42 to any other Aβ variant in a subject with Aβ amyloidosis may be a number other than one 1. In other words, a ratio of relative labeling of Aβ42 to any other Aβ variant in a subject of about 1 indicates the absence of Aβ amyloidosis. Conversely, a ratio of relative labeling of Aβ42 to any other Aβ variant in a subject is other than one 1, indicates the presence of Aβ amyloidosis.

In some embodiments, the ratio of relative labeling of Aβ protein to Aβ42 may be measured. In other embodiments, the ratio of relative labeling of Aβ42 to Aβ protein may be measured. In yet other embodiments, the ratio of relative labeling of Aβ protein to Aβ40 may be measured. In additional embodiments, the ratio of relative labeling of Aβ40 to Aβ protein variant may be measured. In still other embodiments, the ratio of relative labeling of Aβ protein to Aβ38 may be measured. In other embodiments, the ratio of relative labeling of Aβ38 to Aβ protein variant may be measured. In additional embodiments, the ratio of relative labeling of Aβ40 to Aβ38 may be measured. In yet embodiments, the ratio of relative labeling of Aβ38 to Aβ40 may be measured. In still embodiments, the ratio of relative labeling of Aβ42 to Aβ38 may be measured. In other embodiments, the ratio of relative labeling of Aβ38 to Aβ42 may be measured. In some embodiments, the ratio of relative labeling of Aβ40 to Aβ42 may be measured. In preferred embodiments, the ratio of relative labeling of Aβ42 to Aβ40 may be measured.

As described in the examples, the relative labeling of Aβ42 in subjects with Aβ amyloidosis may be higher than the relative labeling of other Aβ variants in CSF samples taken at about 4 to about 22 hours. Therefore, the ratio of relative labeling of Aβ42 to Aβ40 in CSF samples taken at about 4 to about 17 hours may be greater than about 1. In some embodiments, a ratio of relative labeling of Aβ42 to Aβ40 greater than about 1 in a CSF sample taken at about 4 to about 22 hours indicates the presence of Aβ amyloidosis. In other embodiments, a ratio of relative labeling of Aβ42 to Aβ40 greater than about 1 in a CSF sample taken at about 4 to about 17 hours indicates the presence of Aβ amyloidosis. In one embodiment, a ratio of relative labeling of Aβ42 to Aβ40 greater than about 1 in a CSF sample taken at about 5, 6, 7, 8, 9, 10, 11, 12, 13, 14, 15, 16, 17 hours or more, indicates the presence of Aβ amyloidosis. In another embodiment, a ratio of relative labeling of Aβ42 to Aβ40 greater than about 1 in a CSF sample taken at about 8, 9, 10, 11, 12 hours or more, indicates the presence of Aβ amyloidosis. In a preferred embodiment, a ratio of relative labeling of Aβ42 to Aβ40 greater than about 1 in a CSF sample taken at about 10 hours, indicates the presence of Aβ amyloidosis.

In some embodiments, a ratio of relative labeling of Aβ42 to Aβ40 may be about 1.04, 1.05, 1.06, 1.07, 1.08, 1.09, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4 or above in a CSF sample taken at about 4 to about 22 hours, indicating the presence of Aβ amyloidosis. In other embodiments, a ratio of relative labeling of Aβ42 to Aβ40 may be about 1.01, 1.02, 1.03, 1.04, 1.05, 1.06, 1.07, 1.08, 1.09, 1.1, 1.2, 1.3, 1.4, or 1.5 in a CSF sample taken at about 4 to about 17 hours, indicating the presence of Aβ amyloidosis. In yet other embodiments, a ratio of relative labeling of Aβ42 to Aβ40 may be about 1.09, 1.1, 1.2, 1.3, 1.4, 1.5, 1.6, 1.7, 1.8, 1.9, or 2 in a CSF sample taken at about 4 to about 22 hours, indicating the presence of Aβ amyloidosis. In additional embodiments, a ratio of relative labeling of Aβ42 to Aβ40 may be about 1.5, 1.6, 1.7, 1.8, 1.9, 2, 2.1, 2.2, 2.3, 2.4, 2.5, 2.6, 2.7, 2.8, 2.9, or 3.0 in a CSF sample taken at about 4 to about 22 hours, indicating the presence of Aβ amyloidosis. In other embodiments, a ratio of relative labeling of Aβ42 to Aβ40 may be about 2.5, 2.6, 2.7, 2.8, 2.9, 3.0, 3.1, 3.2, 3.3, 3.4, 3.5, 3.6, 3.7, 3.8, 3.9, 4 or above in a CSF sample taken at about 4 to about 22 hours, indicating the presence of Aβ amyloidosis.

In some embodiments, a ratio of relative labeling of Aβ42 to Aβ40 of about 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, or above in a CSF sample taken at about 6 hours, indicates the presence of Aβ amyloidosis. In other embodiments, a ratio of relative labeling of Aβ42 to Aβ40 of about 1.4, 1.5, 1.6, 1.7, 1.8, 1.9, 2, 2.1, 2.2, 2.3, 2.4, 2.5, or above in a CSF sample taken at about 6 hours, indicates the presence of Aβ amyloidosis. In yet other embodiments, a ratio of relative labeling of Aβ42 to Aβ40 of about 1.4, 1.41, 1.42, 1.43, 1.44, 1.45, 1.46, 1.47, 1.48, 1.49, 1.5,, 1.51, 1.52, 1.53, 1.54, 1.55, 1.56, 1.57, 1.58, 1.59, 1.6, 1.61, 1.62, 1.63, 1.64, 1.65, 1.66, 1.67, 1.68, 1.69, 1.7, 1, 1, 1.72, 1.73, 1.74, 1.75, 1.76, 1.77, 1.78, 1.79, 1.8, 1.81, 1.82, 1.83, 1.84, 1.85, 1.86, 1.87, 1.88, 1.89, 1.9, 1.91, 1.92, 1.93, 1.94, 1.95, 1.96, 1.97, 1.98, 1.99, 2, 2.1, 2.1, 2.3, 2.4, 2.5, or above in a CSF sample taken at about 6 hours, indicates the presence of Aβ amyloidosis.

In some embodiments, a ratio of relative labeling of Aβ42 to Aβ40 of about 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, or above in a CSF sample taken at about 7 hours, indicates the presence of Aβ amyloidosis. In other embodiments, a ratio of relative labeling of Aβ42 to Aβ40 of about 1.4, 1.5, 1.6, 1.7, 1.8, 1.9, 2, 2.1, 2.2, 2.3, 2.4, 2.5, or above in a CSF sample taken at about 7 hours, indicates the presence of Aβ amyloidosis. In yet other embodiments, a ratio of relative labeling of Aβ42 to Aβ40 of about 1.4, 1.41, 1.42, 1.43, 1.44, 1.45, 1.46, 1.47, 1.48, 1.49, 1.5,, 1.51, 1.52, 1.53, 1.54, 1.55, 1.56, 1.57, 1.58, 1.59, 1.6, 1.61, 1.62, 1.63, 1.64, 1.65, 1.66, 1.67, 1.68, 1.69, 1.7, 1, 1, 1.72, 1.73, 1.74, 1.75, 1.76, 1.77, 1.78, 1.79, 1.8, 1.81, 1.82, 1.83, 1.84, 1.85, 1.86, 1.87, 1.88, 1.89, 1.9, 1.91, 1.92, 1.93, 1.94, 1.95, 1.96, 1.97, 1.98, 1.99, 2, 2.1, 2.1, 2.3, 2.4, 2.5, or above in a CSF sample taken at about 7 hours, indicates the presence of Aβ amyloidosis.

In some embodiments, a ratio of relative labeling of Aβ42 to Aβ40 of about 1.2, 1.3, 1.4, 1.5, 1.6, 1.7, 1.8, 1.9, 2, 2,1, 2.2, 2.3, 2.4, 2.5, or above in a CSF sample taken at about 8 hours, indicates the presence of Aβ amyloidosis. In other embodiments, a ratio of relative labeling of Aβ42 to Aβ40 of about 1.2, 1.21, 1.22, 1.23, 1.24, 1.25, 1.26, 1.27, 1.28, 1.29, 1.3, 1.31, 1.32, 1.33, 1.34, 1.35, 1.36, 1.37, 1.38, 1.39, 1.4, 1.41, 1.42, 1.43, 1.44, 1.45, 1.46, 1.47, 1.48, 1.49, 1.5,, 1.51, 1.52, 1.53, 1.54, 1.55, 1.56, 1.57, 1.58, 1.59, 1.6, 1.61, 1.62, 1.63, 1.64, 1.65, 1.66, 1.67, 1.68, 1.69, 1.7, 1, 1, 1.72, 1.73, 1.74, 1.75, 1.76, 1.77, 1.78, 1.79, 1.8, 1.81, 1.82, 1.83, 1.84, 1.85, 1.86, 1.87, 1.88, 1.89, 1.9, 1.91, 1.92, 1.93, 1.94, 1.95, 1.96, 1.97, 1.98, 1.99, 2, 2.1, 2.1, 2.3, 2.4, 2.5, or above in a CSF sample taken at about 8 hours, indicates the presence of Aβ amyloidosis.

In some embodiments, a ratio of relative labeling of Aβ42 to Aβ40 of about 1.2, 1.3, 1.4, 1.5, 1.6, 1.7, 1.8, 1.9, 2, 2,1, 2.2, 2.3, 2.4, 2.5, or above in a CSF sample taken at about 9 hours, indicates the presence of Aβ amyloidosis. In other embodiments, a ratio of relative labeling of Aβ42 to Aβ40 of about 1.2, 1.21, 1.22, 1.23, 1.24, 1.25, 1.26, 1.27, 1.28, 1.29, 1.3, 1.31, 1.32, 1.33, 1.34, 1.35, 1.36, 1.37, 1.38, 1.39, 1.4, 1.41, 1.42, 1.43, 1.44, 1.45, 1.46, 1.47, 1.48, 1.49, 1.5,, 1.51, 1.52, 1.53, 1.54, 1.55, 1.56, 1.57, 1.58, 1.59, 1.6, 1.61, 1.62, 1.63, 1.64, 1.65, 1.66, 1.67, 1.68, 1.69, 1.7, 1, 1, 1.72, 1.73, 1.74, 1.75, 1.76, 1.77, 1.78, 1.79, 1.8, 1.81, 1.82, 1.83, 1.84, 1.85, 1.86, 1.87, 1.88, 1.89, 1.9, 1.91, 1.92, 1.93, 1.94, 1.95, 1.96, 1.97, 1.98, 1.99, 2, 2.1, 2.1, 2.3, 2.4, 2.5, or above in a CSF sample taken at about 9 hours, indicates the presence of Aβ amyloidosis.

In some preferred embodiments, a ratio of relative labeling of Aβ42 to Aβ40 of about 1.1, 1.2, 1.3, 1.4, 1.5, 1.6, 1.7, 1.8, 1.9, 2 or above in a CSF sample taken at about 10 hours, indicates the presence of Aβ amyloidosis. In other preferred embodiments, a ratio of relative labeling of Aβ42 to Aβ40 of about 1.2, 1.21, 1.22, 1.23, 1.24, 1.25, 1.26, 1.27, 1.28, 1.29, 1.3, 1.31, 1.32, 1.33, 1.34, 1.35, 1.36, 1.37, 1.38, 1.39, 1.4, 1.41, 1.42, 1.43, 1.44, 1.45, 1.46, 1.47, 1.48, 1.49, 1.5,, 1.51, 1.52, 1.53, 1.54, 1.55, 1.56, 1.57, 1.58, 1.59, 1.6, 1.61, 1.62, 1.63, 1.64, 1.65, 1.66, 1.67, 1.68, 1.69, 1.7, 1, 1, 1.72, 1.73, 1.74, 1.75, 1.76, 1.77, 1.78, 1.79, 1.8, 1.81, 1.82, 1.83, 1.84, 1.85, 1.86, 1.87, 1.88, 1.89, 1.9, 1.91, 1.92, 1.93, 1.94, 1.95, 1.96, 1.97, 1.98, 1.99, 2 or above in a CSF sample taken at about 10 hours, indicates the presence of Aβ amyloidosis. In yet other preferred embodiments, a ratio of relative labeling of Aβ42 to Aβ40 of about 1.25, 1.26, 1.27, 1.28, 1.29, 1.3, 1.31, 1.32, 1.33, 1.34, 1.35, 1.36, 1.37, 1.38, 1.39, 1.4, 1.41, 1.42, 1.43, 1.44, 1.45, 1.46, 1.47, 1.48, 1.49, 1.5,, 1.51, 1.52, 1.53, 1.54, 1.55, 1.56, 1.57, 1.58, 1.59, 1.6, 1.61, 1.62, 1.63, 1.64, 1.65, 1.66, 1.67, 1.68, 1.69, or 1.7 in a CSF sample taken at about 10 hours, indicates the presence of Aβ amyloidosis. In yet other preferred embodiments, a ratio of relative labeling of Aβ42 to Aβ40 of about 1.3, 1.31, 1.32, 1.33, 1.34, 1.35, 1.36, 1.37, 1.38, 1.39, 1.4, 1.41, 1.42, 1.43, 1.44, 1.45, 1.46, 1.47, 1.48, 1.49, 1.5,, 1.51, 1.52, 1.53, 1.54, 1.55, 1.56, 1.57, 1.58, 1.59, 1.6, 1.61, 1.62, 1.63, 1.64, 1.65 in a CSF sample taken at about 10 hours, indicates the presence of Aβ amyloidosis.

As described in the examples, the relative labeling of the Aβ42 variant in subjects with Aβ amyloidosis may be lower than the relative labeling of other Aβ variants in CSF samples taken at about 22 to about 32 hours. Therefore, the ratio of relative labeling of Aβ42 to Aβ40 in CSF samples taken at about 22 to about 32 hours may be lower than about 1.

In some embodiments, a ratio of relative labeling of Aβ42 to Aβ40 less than about 1 in a CSF sample taken at about 22 to about 32 hours indicates the presence of Aβ amyloidosis. In one embodiment, a ratio of relative labeling of Aβ42 to Aβ40 less than about 1 in a CSF sample taken at about 22, 23, 24, 25, 26, 27, 28, 29, 30, 31, or 32 hours or more, indicates the presence of Aβ amyloidosis. In another embodiment, a ratio of relative labeling of Aβ42 to Aβ40 less than about 1 in a CSF sample taken at about 25, 26, 27, 28, 29 hours or more indicates the presence of Aβ amyloidosis. In yet another embodiment, a ratio of relative labeling of Aβ42 to Aβ40 less than about 1 in a CSF sample taken at about 23, 24, 25, 26, 27, 28, 29, 30, 31 hours or more indicates the presence of Aβ amyloidosis.

In some embodiments, a ratio of relative labeling of Aβ42 to Aβ40 less than about 1 in a CSF sample taken at about 23 hours indicates the presence of Aβ amyloidosis. In one embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 23 hours, indicating the presence of Aβ amyloidosis. In another embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 23 hours, indicating the presence of Aβ amyloidosis.

In some embodiments, a ratio of relative labeling of Aβ42 to Aβ40 less than about 1 in a CSF sample taken at about 24 hours indicates the presence of Aβ amyloidosis. In one embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 24 hours, indicating the presence of Aβ amyloidosis. In another embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 24 hours, indicating the presence of Aβ amyloidosis.

In some embodiments, a ratio of relative labeling of Aβ42 to Aβ40 less than about 1 in a CSF sample taken at about 25 hours indicates the presence of Aβ amyloidosis. In one embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 25 hours, indicating the presence of Aβ amyloidosis. In another embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 25 hours, indicating the presence of Aβ amyloidosis.

In some embodiments, a ratio of relative labeling of Aβ42 to Aβ40 less than about 1 in a CSF sample taken at about 26 hours indicates the presence of Aβ amyloidosis. In one embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 26 hours, indicating the presence of Aβ amyloidosis. In another embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 26 hours, indicating the presence of Aβ amyloidosis.

In some embodiments, a ratio of relative labeling of Aβ42 to Aβ40 less than about 1 in a CSF sample taken at about 28 hours indicates the presence of Aβ amyloidosis. In one embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 28 hours, indicating the presence of Aβ amyloidosis. In another embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 28 hours, indicating the presence of Aβ amyloidosis.

In some embodiments, a ratio of relative labeling of Aβ42 to Aβ40 less than about 1 in a CSF sample taken at about 29 hours indicates the presence of Aβ amyloidosis. In one embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 29 hours, indicating the presence of Aβ amyloidosis. In another embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 29 hours, indicating the presence of Aβ amyloidosis.

In some embodiments, a ratio of relative labeling of Aβ42 to Aβ40 less than about 1 in a CSF sample taken at about 30 hours indicates the presence of Aβ amyloidosis. In one embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 30 hours, indicating the presence of Aβ amyloidosis. In another embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 30 hours, indicating the presence of Aβ amyloidosis.

In some embodiments, a ratio of relative labeling of Aβ42 to Aβ40 less than about 1 in a CSF sample taken at about 31 hours indicates the presence of Aβ amyloidosis. In one embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 31 hours, indicating the presence of Aβ amyloidosis. In another embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 31 hours, indicating the presence of Aβ amyloidosis.

In preferred embodiments, a ratio of relative labeling of Aβ42 to Aβ40 less than about 1 in a CSF sample taken at about 27 hours indicates the presence of Aβ amyloidosis. In a preferred embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a CSF sample taken at about 27 hours, indicating the presence of Aβ amyloidosis. In another preferred embodiment, a ratio of relative labeling of Aβ42 to Aβ40 may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a CSF sample taken at about 27 hours, indicating the presence of Aβ amyloidosis.

In a preferred embodiment, a ratio of relative labeling of Aβ42 to Aβ40 less than about 1 in a CSF sample taken at about 27 hours indicates the presence of Aβ amyloidosis. In yet another preferred embodiment, a ratio of relative labeling of Aβ42 to Aβ40 may be about 0.7, 0.6, 0.5, 0.4, or about 0.3 in a CSF sample taken at about 27 hours, indicating the presence of Aβ amyloidosis.

The relative labeling of the Aβ42 variant in subjects with Aβ amyloidosis may be higher than the relative labeling of other Aβ variants in a blood sample taken at about 1 to about 32 hours. Therefore, a ratio of relative labeling of Aβ42 to another Aβ variant in a blood sample taken at about 1 to about 32 hours may be more than about 1. In some embodiments, a ratio of relative labeling of Aβ42 to another Aβ variant may be more than about 1 in a blood sample taken at about 1 to about 32 hours, indicating the presence of Aβ amyloidosis. In one embodiment, a ratio of relative labeling of Aβ42 to another Aβ variant may be more than about 1 in a blood sample taken at about 1 to about 4 hours, such as about 1, 2, 3, or 4 hours or more, indicating the presence of Aβ amyloidosis. In another embodiment, a ratio of relative labeling of Aβ42 to another Aβ variant may be more than about 1 in a blood sample taken at about 10 min, 15 min, 30 min, 1 hour, 90 min, 2 hours, 2.5 hours, 3 hours, or 3.5 hours, indicating the presence of Aβ amyloidosis. In still another embodiment, a ratio of relative labeling of Aβ42 to another Aβ variant may be more than about 1 in a blood sample taken between about 10 min to 60 min, 30 min to 90 min, 60 min to 2 hours, 90 min to 2.5 hours, or 2 hours to 3 hours, indicating the presence of Aβ amyloidosis.

In some embodiments, a ratio of relative labeling of Aβ42 to an Aβ variant may be about 1.04, 1.05, 1.06, 1.07, 1.08, 1.09, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4 or above in a blood sample taken between about 0 to about 3 hours after labeling, indicating the presence of Aβ amyloidosis. In other embodiments, a ratio of relative labeling of Aβ42 to an Aβ variant may be about 1.01, 1.02, 1.03, 1.04, 1.05, 1.06, 1.07, 1.08, 1.09, 1.1, 1.2, 1.3, 1.4, or 1.5 in a blood sample taken between about 0 to about 3 hours, indicating the presence of Aβ amyloidosis. In yet other embodiments, a ratio of relative labeling of Aβ42 to an Aβ variant may be about 1.09, 1.1, 1.2, 1.3, 1.4, 1.5, 1.6, 1.7, 1.8, 1.9, or 2 in a blood sample taken between about 0 to about 4 hours, indicating the presence of Aβ amyloidosis. In additional embodiments, a ratio of relative labeling of Aβ42 to an Aβ variant may be about 1.5, 1.6, 1.7, 1.8, 1.9, 2, 2.1, 2.2, 2.3, 2.4, 2.5, 2.6, 2.7, 2.8, 2.9, or 3.0 in a blood sample taken between about 0 to about 3 hours, indicating the presence of Aβ amyloidosis. In other embodiments, a ratio of relative labeling of Aβ42 to an Aβ variant may be about 2.5, 2.6, 2.7, 2.8, 2.9, 3.0, 3.1, 3.2, 3.3, 3.4, 3.5, 3.6, 3.7, 3.8, 3.9, 4 or above in a blood sample taken between about 0 to about 3 hours, indicating the presence of Aβ amyloidosis.

In some embodiments, a ratio of relative labeling of Aβ42 to another Aβ variant may be less than about 1 in blood samples taken between about 4.5 hours to about 10 hours, indicating the presence of Aβ amyloidosis. In one embodiment, a ratio of relative labeling of Aβ42 to another Aβ variant may be less than about 1 in blood samples taken at about 4, 5, 6, 7, 8, 9, 10, 11, 12, 13, 14, 15, 16, 17, 18, 19, 20, 21, 22, 23, 24, 25, 26, 27, 28, 29, 30, 31, or 32 hours or more after labeling, indicating the presence of Aβ amyloidosis. In another embodiment, a ratio of relative labeling of Aβ42 to another Aβ variant may be less than about 1 in blood samples taken at about 4, 4.5, 5, 5.5, 6, 6.5, 7, 7.5, 8, 8.5, 9, or 9.5 hours or more, indicating the presence of Aβ amyloidosis.

In some embodiments, a ratio of relative labeling of Aβ42 to an Aβ variant of less than about 1 in a blood sample taken between about 5 to about 9 hours after labeling indicates the presence of Aβ amyloidosis. In one embodiment, a ratio of relative labeling of Aβ42 to an Aβ variant may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5, 0.49, 0.48, 0.47, 0.46, 0.45, 0.44, 0.43, 0.42, 0.41, 0.4, 0.39, 0.38, 0.37, 0.36, 0.35, 0.34, 0.33, 0.32, 0.31, 0.3, 0.29, 0.28, 0.27, 0.26, 0.25, 0.24, 0.23, 0.22, 0.21, 0.2, 0.1, or less in a blood sample taken at about 5 to about 9 hours, indicating the presence of Aβ amyloidosis. In another embodiment, a ratio of relative labeling of Aβ42 to an Aβ variant may be about 0.9, 0.89, 0.88, 0.87, 0.86, 0.85, 0.84, 0.83, 0.82, 0.81, 0.8, 0.79, 0.78, 0.77, 0.76, 0.75, 0.74, 0.73, 0.72, 0.71, 0.7, 0.69, 0.68, 0.67, 0.66, 0.65, 0.64, 0.63, 0.62, 0.61, 0.6, 0.59, 0.58, 0.57, 0.56, 0.55, 0.54, 0.53, 0.52, 0.51, 0.5 or less in a blood sample taken at about 5 to about 9 hours, indicating the presence of Aβ amyloidosis.

In one exemplary embodiment, the *in vivo* relative labeling of Aβ42 and Aβ40 are measured by administering labeled leucine to a subject and collecting one or more biological samples at regular intervals over 36 hours. The biological sample may be collected from blood plasma or CSF. The amount of labeled and unlabeled Aβ in the biological samples is typically determined by immunoprecipitation followed by LC-ESI-tandem MS. From these measurements, a ratio of labeled to unlabeled Aβ may be calculated to determine the relative labeling of Aβ42 and Aβ40. A ratio of relative labeling of Aβ42 to relative labeling of Aβ40 may then be calculated in a given biological sample. A ratio of relative labeling of Aβ42 to relative labeling of Aβ40 in a given sample other than 1 indicates the presence of Aβ amyloidosis.

### II. Kits for diagnosing or monitoring the progression or treatment of neurological and neurodegenerative diseases

Described are kits for diagnosing or monitoring the progression or treatment of Aβ amyloidosis by measuring the ratio of relative labeling of two Aβ variants in a subject. Generally, a kit comprises a labeled amino acid, means for administering the labeled amino acid, means for collecting biological samples over time, and instructions for detecting and determining the ratio of labeled to unlabeled protein so that the ratio of relative labeling of the two Aβ variants may be calculated. A ratio of relative labeling of the two Aβ variants of about one indicates the absence of Aβ amyloidosis, whereas a ratio of relative labeling of the two Aβ variants other than one indicates the absence of Aβ amyloidosis. These comparisons may enable a practitioner to predict the advent of AD, diagnose the onset of AD, monitor the progression of Aβ amyloidosis, or verify the effectiveness of a treatment for Aβ amyloidosis. In a preferred embodiment, the kit comprises ¹³C₆-leucine or ¹³C₆-phenylalanine, the Aβ variants to be measued are Aβ42 and Aβ40, and the disease to be assessed is AD.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

"Isotope" refers to all forms of a given element whose nuclei have the same atomic number but have different mass numbers because they contain different numbers of neutrons. By way of a non-limiting example, ¹²C and ¹³C are both stable isotopes of carbon.

"Lag time" generally refers to the delay of time from when the biomolecule is first labeled until the labeled biomolecule is detected.

"Metabolism" refers to any combination of the synthesis, transport, breakdown, modification, or clearance rate of a biomolecule.

"Metabolic index" refers to a measurement comprising the fractional synthesis rate (FSR) and the fractional clearance rate (FCR) of the biomolecule of interest. Comparison of metabolic indices from normal and diseased individuals may aid in the diagnosis or monitoring of neurological or neurodegenerative diseases.

"Neurally derived cells" includes all cells within the blood-brain-barrier including neurons, astrocytes, microglia, choroid plexus cells, ependymal cells, other glial cells, etc.

"Steady state" refers to a state during which there is insignificant change in the measured parameter over a specified period of time.

"Synthesis rate" refers to the rate at which the biomolecule of interest is synthesized.

In metabolic tracer studies, a "stable isotope" is a nonradioactive isotope that is less abundant than the most abundant naturally occurring isotope.

"Subject" as used herein means a living organism having a central nervous system. In particular, the subject is a mammal. Suitable subjects include research animals, companion animals, farm animals, and zoo animals. The preferred subject is a human.

### EXAMPLES

The following examples are included to demonstrate preferred embodiments of the invention.

### Example 1. Aβ turnover in blood

Genetic, biochemical, and animal model studies strongly support the hypothesis that amyloid-β (Aβ) plays a central role in AD: more specifically that accumulation and conformational change of Aβ in toxic forms are major contributors to AD pathogenesis.

A pioneering approach was recently developed to directly measure Aβ metabolism in the central nervous system of living humans. This method requires participants be admitted to a research hospital room, and have two IV catheters and a lumbar spinal catheter placed so that hourly samples of blood and cerebral-spinal fluid can be obtained. Using this method, recent studies have demonstrated that Aβ has a rapid metabolism (half-life of 8-10 hours) in the human brain and cerebral-spinal fluid (CSF). We have recently measured the dose-related effects of a proposed disease modifying treatment for AD, a gamma-secretase inhibitor, and have demonstrated direct inhibition of the production of Aβ in the human central nervous system. In addition, preliminary data suggest that patients with even very mild AD have disturbed Aβ metabolism in the central nervous system versus age matched controls.

These findings are important to the understanding of the causes of AD and the development of treatments; however, blood Aβ dynamics are not well understood as there previously was not yet a method to measure labeled Aβ within blood. If such a method were available, the physiology (and pathophysiology) of Aβ could be better understood as a quantitative measure of Aβ production in the brain, transport to blood and cerebral-spinal fluid, and clearance from blood. A blood labeled Aβ assay would allow for the physiology and pathophysiology of Aβ to be measured without invasive spinal catheters, and also allow for the large scale investigation of a diagnostic test for AD, and specific testing of proposed disease modifying therapeutics which target Aβ.

A stable isotope labeling kinetics immunoprecipitation-mass spectrometry approach was developed to measure labeled blood Aβ. This provides the ability to measure blood Aβ production, transport between compartments, and clearance rates in humans.

The half-life of blood/plasma Aβ is distinctly different than in the CNS/CSF, with a t1/2 of 2 to 3 hours in production (**FIG. 1**). This contrasts with a half-life of 9 to 10 hours as measured in CSF (Bateman et. al 2006). For **FIG. 1****,** subjects were administered a labeled amino acid (^{13C6} leucine; infused over 9 hours with a 10 minute primed infusion of 2 mg/kg, followed by 2 mg/kg/hour for 8 hours and 50 minutes) and then blood samples were taken every hour for 0 - 15 hr, then every odd hour till hour 35, then at 36 & 48 hours. Twenty-eight samples were taken in total. The samples were stored frozen. Then, samples were thawed and a protease inhibitor cocktail was added. Samples were centrifuged and then pooled from two samples of plasma (2 mls total) at every time point. Next, Aβ was immunoprecipitated from the samples as described below:
Day 1:
   1. Thaw samples slowly on ice (approx 1 hr).
   2. When thawed, add 20 µl of Complete Protease Inhibitors
   3. Centrifuge at 14,000 RPM in the Allegra centrifuge at 4°C for 15 minutes to remove particulates.
   4. Pool the supernatant from 2 samples together (2 ml in 15 ml tubes).
   5. Dilute media standards - 50 µl into 2 ml PBS.
   6. Wash HJ5.1 (Aβ specific antibody) beads twice with 1x PBS with 0.02% azide. Make 50% bead slurry at the end.
   7. Add 220 µl 5M guanidine hydrochloride solution.
   8. Add 20 µl Tween-20 (5% in PBS for 0.05% final)
   9. Add 5 µl of ISTD (N15- Aβ 40/42).
   10. Add 30 µl antibody- beads (50% slurry enough for ∼20ng Aβ).
   11. Incubate overnight at 4°C.
Day 2:
   12. Centrifuge beads at 4500 RPM for 5 minutes.
   13. Remove supernatant into a new tube and save (plasma only).
   14. Add 1 ml 0.5M guanidine hydrochloride solution
   15. Add 10 µl of 5% for 0.05% final Tween-20.
   16. Once re-suspended transfer beads to 1.5 ml Axygen tube.
   17. Centrifuge beads at 4500 RPM for 5 minutes.
   18. Discard supernatant using the aspirator-vacuum system, using a crimped pipette tip.
   19. Wash the beads two times as follows:
      a. 1ml 1X AmBiC rinse
      b. 4500 RPM for 5 minutes
      c. Discard supernatant
      d. 1ml 1XAmBiC rinse
      e. 4500 RPM for 5 minutes
      f. Discard the supernant and dry beads
   20. Add 50 µl (bead volume) 98% formic acid (elution solution) and centrifuge beads at 4500 RPM for 2 minutes and remove supernatant with crimped filter gel loading tip and place in 1.5 ml Axygen tubes.
   21. Dry the elution solution in a speedvac for 1 hour to remove formic acid (37°C, 60 min, 60 min).
   22. Make 25mM AmBiC solution by with HPLC grade water.
   23. Add 1 ml of 25mM AmBiC solution to trypsin to a final concentration of 20 ng/µl.
   24. Re-suspend the proteins in 10 µl solution of 25 mM AmBiC.
   25. Add 10 µl of the 20 ng/µl trypsin solution to the beads for digestion.
   26. Digest O/N (16 hrs) at 37°C in the incubator.
DAY 3:
   27. Perform a short spin of the samples to let condensate settle at the bottom of tube.
   28. Add 2 µl of formic acid to the digest (to precipitate proteins)
   29. Place the rack of samples on the vortex and mix for 30 seconds.
   30. Centrifuge the tryptic digest at 14,000 RPM in the Allegra centrifuge at 4°C for 15 minutes.
   31. Transfer to autosampler vials then spin down samples using the speedvac and keep in the refrigerator at 4°C.

The samples were then analyzed by mass spectrometry as follows:

The amino acid sequence of Aβ 1-42 is DAEFRHDSGYEVHH QKLVFFAEDVGSNKGAIIGLMVGGVVIA (SEQ ID NO:1), Aβ 1-40 is DAEFRHDSGYEVHHQKLVFFAEDVGSNKGAIIGLMVGGVV (SEQ ID NO:2), and Aβ 1-38 is DAEFRHDSGYEVHHQKLVFFAEDVGS NKGAIIGLMVGG (SEQ ID NO:3). When it is digested with a protease like trypsin, four smaller peptides are generated as Aβ 1-5 DAEFR(SEQ ID NO:4), Aβ 6-16 HDSGYEVHHQK(SEQ ID NO:5), Aβ 17-28 LVFFAEDVGSNK(SEQ ID NO:6), and Aβ 29-42 GAIIGLMVGGVVIA(SEQ ID NO:7). Only two of these peptides are of relevance for quantitation using the SILK technology (Stable Isotope Labeling Kinetics - Bateman et. al 2009) with leucine labeling because their sequence includes a leucine residue that can be labeled in vivo. These peptides are Aβ 17-28 LVFFAEDVGSNK(SEQ ID NO:6), and Aβ 29-42 GAIIGLMVGGVVIA(SEQ ID NO:7), but we use only the Aβ 17-28 LVFFAEDVGSNK(SEQ ID NO:6) peptide because it represents the total amounts of Aβ in the blood and produces a higher signal. Of course, it may be useful to also quantify the c-terminal (Aβ 29-42 GAIIGLMVGGVVIA (SEQ ID NO:7) or Aβ 29-40 GAIIGLMVGGVV(SEQ ID NO:8)) or n-terminal fragments in the blood as well. In general, any amino acid label can be used, any endoprotease can be used (or none at all) to label Aβ & quantify the label in the blood. The blood Aβ metabolism may directly reflect a disequilibrium of Aβ metabolism that indicates Alzheimer's disease or predisposition to Alzheimer's disease.

Experiments were performed on a TSQ Vantage mass spectrometer coupled to a nanoflow ESI source and a nano-2D liquid chromatography (NanoLC-2D). The samples were maintained at a temperature of 4°C in the autosampler tray. Samples were injected onto a home-made nano column (150-mm diameter) with a pulled tip, packed to 15 cm with Ace 5 C18 AR column packing material (MAC-MOD Analytical, Chadds Ford, PA). Peptides were separated by the Nano2D-LC (Eksigent, Inc. Ultra NanoLC-2D) at a flow rate of 1 mL/ min. Solvent A was 0.1 % formic acid in water and solvent B was 0.1 % formic acid in acetonitrile. The gradient was 15% B to 25% B in 10 min followed by 25% B to 95% B in 5 min, then, ramped down to 15% B in 2 min and the column re-equilibrated for 3 min while the autosampler was picking another sample for injection.

The TSQ Vantage mass spectrometer (MS) was operated in positive ion mode using a spray voltage of 1.2 kV, with optimized parameters from tuning with peptides. Data was acquired in Multiple Reaction Monitoring (MRM) mode. During this MRM experiments, the mass of the peptide was first detected in the first dimension, or as MS1. While MS1 is used to perform quantitation the technique suffers from lack of specificity especially in very complex matrices like blood and that many peptides have the same intact mass. The peptide ions were fragmented and detected in the mass spectrometer and this second dimension of MS fragmentation (MS2) provided unique fragments. The combination of the specific precursor mass and the unique fragment ions were used to selectively monitor the peptide to be quantified. In this case, multiple fragment ions (also known as reactions) were selectively monitored to quantitate Aβ 17-28, resulting in a unique MRM experiment. Aβ 17-28 has a precursor mass (MS1) with a charge to mass ratio of 663.340 for the endogenous peptide and 666.340 for the label incorporated peptide. Three each of their fragment ions were monitored after the MS2 fragmentation. The fragment ions also known as transition ions monitored have a mass to charge ratios of 819.38, 966.45 and 1113.52. The MRM experiments are detected and plotted as single chromatographic peaks which were processed by Xcalibur, which is the instrument control software.

Results are described in **FIG. 2****.** Note the rapid rise to plateau by 9 hours with a rapid clearance rate in blood, while CSF does not approach plateau until 18 hours or later. Also note the much more rapid clearance of blood Aβ compared to CSF Aβ. There also may be a second peak of labeled Aβ in blood from 20 to 30 hours (peak ∼26 hours).

### Example 2. Aβ blood kinetics

In order to determine blood Aβ kinetics, a method was developed to measure ¹³C₆-leucine labeled Aβ in plasma. Prior studies in animal models demonstrate faster Aβ turnover in the periphery compared to the CNS. The new method required a state-of-the-art mass spectrometer with levels of quantitation in the low attomole (10⁻¹⁸) range. The first measurements of blood Aβ kinetics are shown compared to CSF Aβ kinetics in **FIG. 3****.**

These results demonstrate blood Aβ kinetics are distinct from CNS Aβ kinetics and can be utilized in multi-compartment models to calculate the rates of formation, transport, and breakdown of Aβ both in the CNS and body.

In order to compare the kinetics of blood Aβ in AD versus controls, we measured labeled plasma Aβ in blood samples from 12AD and 8 control participants and compared as shown in **FIG. 4****.**

In order to provide key additional kinetic information on blood Aβ kinetics which has a much faster turnover rate, and to potentially better distinguish AD from controls, an alternative oral pulse labeling protocol was developed (**FIG. 5**). The pulse labeling provides additional information on Aβ transport and clearance and enables evaluation of the secondary slower clearance rates that can distinguish models of Aβ transport. Further, the pulse oral labeling method is simplified as a single oral drink to enable straightforward implementation of blood Aβ kinetics as a clinical test for treatment trials or as a diagnostic test.

To expand understanding of specific Aβ variants which may be uniquely altered in AD (e.g. Aβ42), we have developed a method to measure Aβ c-terminal variants in the same sample (**FIG. 6**). This novel quantitative approach allows us to evaluate the major Aβ variants in the same sample.

### Example 3. Decreased clearance of CNS β-amyloid in Alzheimer's disease

Alzheimer's disease (AD) is characterized by increased amounts of soluble and insoluble β-amyloid (Aβ), predominantly in the form of Aβ42 in amyloid plaques and Aβ40 in amyloid angiopathy. The amyloid hypothesis proposes that AD is caused by an imbalance between Aβ production and clearance (1), resulting in increased amounts of Aβ in various forms such as monomers, oligomers, insoluble fibrils, and plaques in the central nervous system (CNS). High levels of Aβ then initiate a cascade of events culminating in neuronal damage and death manifesting as progressive clinical dementia of the Alzheimer's type (2).

In rare cases of AD, genetic alterations increase the production of Aβ (3). However, Aβ dysregulation in the far more common late-onset "sporadic" AD is less well understood. Possible mechanisms of increased Aβ production for late-onset AD include alterations in gamma or beta secretase activity. Alternatively, impaired clearance of Aβ may also cause late-onset AD through interactions with ApoE4, decreased catabolism of Aβ via reduced proteolysis, impaired transport across the blood-brain barrier, or impaired cerebrospinal fluid (CSF) transport.

To measure the production and clearance of Aβ in AD, we developed a method to measure human CNS Aβ production and clearance (4) and compared Aβ42 and Aβ40 production and clearance rates in individuals with symptomatic AD and in cognitively normal persons to determine whether either or both are altered in AD.

We plotted the average time course results of labeled Aβ42 and Aβ40 for the production phase (hours 5 to 14) and the clearance phase (hours 24 to 36) (**FIG. 7**). The production and clearance rates were calculated for each participant and compared by group status (AD versus control). The average Aβ42 production rate did not differ between the control (6.7%/hour) and AD (6.6%/hour) groups (P = 0.96), nor did Aβ40 production rate differ between groups (6.8%/hour for controls and 6.8%/hour for the AD group; P = 0.98). The average clearance rate of Aβ42 was slower for AD individuals compared with that for cognitively normal controls (5.3%/hour versus 7.6%/hour, P = 0.03), as was the average clearance rate of Aβ40 (5.2%/hour for AD individuals versus 7.0%/hour for controls; P = 0.01)

To determine the balance of Aβ production to clearance rates in AD versus controls, we measured the ratios of production to clearance (**FIG. 8**). The ratio of Aβ42 production to clearance rates was balanced for cognitively normal participants (0.95); however, because of decreased clearance in the AD participants, there was an imbalance in the Aβ42 production to clearance ratio (1.35). Similarly, we observed an imbalance in the AD Aβ40 production to clearance ratio (1.37) compared with the ratio in cognitively normal participants (0.99).

The technique of measuring Aβ production and clearance has been used to measure effects of drugs that target Aβ generation, demonstrating decreases in production (5). We found that late-onset AD is associated with a 30% impairment in the clearance of both Aβ42 and Aβ40, indicating that Aβ clearance mechanisms may be critically important in the development of AD (6). Estimates based on a 30% decrease in Aβ clearance rates suggest that brain Aβ accumulates over about 10 years in AD. The impaired clearance of both Aβ40 and Aβ42 is consistent with prior findings of deposition of Aβ40 and Aβ42 in parenchymal amyloid plaques and the substantial deposition of Aβ40 in cerebral amyloid angiopathy in about 80% of cases of AD (7).

Limitations of this study include the relatively small numbers of participants (12 in each group) and the inability to prove causality of impaired Aβ clearance for AD. In addition to decreased CNS Aβ clearance, CSF Aβ42 concentrations are decreased in AD compared with those in controls (**FIG. 9**). Taken together, these may be consistent with decreased Aβ42 clearance (efflux) from the brain to the CSF. However, the relationship between decreased concentrations of CSF Aβ42 and decreased CNS clearance of labeled Aβ (**FIG. 10**) is not fully understood. Additional possibilities include more than one pool of Aβ in CSF, undetected pools of Aβ in CSF by enzyme-linked immunosorbent assay (e.g., oligomers), or a combined increase in Aβ production with impaired efflux from parenchyma to CSF. Overall, these results suggest impaired metabolism of Aβ in AD compared with that in controls.

### Materials and methods for Example 3

Research participants were enrolled from the Washington University Alzheimer's Disease Research Center. The inclusion criteria were age >60 years, cognitively normal (Clinical Dementia Rating or CDR 0), very mild AD (CDR 0.5) or mild AD (CDR 1). Exclusion criteria were other primary diagnosis of dementia or significant medical co-morbidity including stroke, bleeding diathesis, anticoagulation, and active infectious process. Full verbal and written informed consent was obtained from all participants. Twenty-four participants were clinically evaluated and rated as either very mild to mild dementia of the Alzheimer type (n=12, average age 77 +/- 7.3 years, range 61 to 85; Apolipoprotein E (ApoE) genotype 4/4 n=2, 3/4 n=4, 3/3 n=5, 2/3 n=1; gender 9 men, 3 women), or as cognitively normal (n=12 average age 70.6 years +/- 6.2, range 65 to 84; ApoE genotype 3/4 n=6, 3/3 n=5, 2/3 n=1; gender 4 men, 8 women). Following CSF sampling, three subjects had headaches and one required a blood patch. One participant discontinued the study because of pre-existing confusion at night.

Participants were admitted to the Washington University Clinical Research Unit at 7AM. Two intravenous and one lumbar intrathecal catheters were placed as previously described (Bateman et. al 2006). After baseline blood and CSF samples were collected, a primed bolus of ¹³C₆-Leucine was infused at 2 mg/kg over 10 minutes, followed by 2 mg/kg/hr for the remaining 8 hours and 50 minutes. During and after infusion, CSF and blood samples were collected for a total of 36 hours and frozen at -70°C. Twelve hours after the lumbar catheter was removed, participants were discharged from the research unit.

All samples were processed and measured in a blinded fashion with data results and individual analysis completed before unblinding to participant's disease state. Aβ42 and then Aβ40 were serially immunoprecipitated from CSF samples using c-terminal specific antibodies, 21f12 and 2g3. The purified Aβ was then digested with trypsin and ¹³C₆-leucine abundance in these tryptic fragments quantified using tandem mass spectrometry as previously described (8, 9).

¹³C₆-leucine labeled media enrichment standards and CSF samples were analyzed using the Stable Isotope Labeling Tandem Mass Spectrometry (SILT MS) method (10) using SILTmass (11). Each tandem mass spectrometry scan was searched against the Aβ FASTA sequence database to identify peptides and assign a score. SILTmass summed the signal intensity for tandem mass spectrometry ions that matched the theoretical b and y ions for Aβ tryptic fragments and saved results as part of a pepXML file. A set of pepXML files for each subject was then analyzed to calculate the ratio of labeled Aβ to unlabeled Aβ by dividing the total labeled and unlabeled signal intensities. The percent ¹³C₆-leucine labeled Aβ was calculated as the ratio of ¹³C₆-leucine-Aβ17-28 divided by natural isotope abundance Aβ17-28. Results were exported to Microsoft Excel, normalized by the slope of the standard curve and leucine labeling ratio (¹³C₆-Leu/¹²C₆-Leu) (12).

Calculation of the Fractional Synthesis Rate (FSR) and Fractional Clearance Rate (FCR) were performed as previously described (9) in a blinded fashion to dementia status. After calculations and measurements were made, the dementia status of each participant was used for statistical comparison. Comparison of FSR and FCR between cognitively normal and AD groups were made by two-tailed t-test with significance set at p<0.05 (Graphpad 5.03).

### References for Example 3

1. J. Hardy, D. J. Selkoe, The amyloid hypothesis of Alzheimer's disease: Progress and problems on the road to therapeutics. Science 297, 353 (2002).
2. J. L. Cummings, Alzheimer's disease. N. Engl. J. Med. 351, 56 (2004).
3. D. Scheuner et al., Secreted amyloid beta-protein similar to that in the senile plaques of Alzheimer's disease is increased in vivo by the presenilin 1 and 2 and APP mutations linked to familial Alzheimer's disease. Nat. Med. 2, 864 (1996).
4. R. J. Bateman et al., Human amyloid-beta synthesis and clearance rates as measured in cerebrospinal fluid in vivo. Nat. Med. 12, 856 (2006).
5. R. J. Bateman et al., A gamma-secretase inhibitor decreases amyloid-beta production in the central nervous system. Ann. Neurol. 66, 48 (2009).
6. R. B. DeMattos et al., ApoE and clusterin cooperatively suppress Abeta levels and deposition: evidence that ApoE regulates extracellular Abeta metabolism in vivo. Neuron 41, 193 (2004).
7. R. J. Ellis et al., Cerebral amyloid angiopathy in the brains of patients with Alzheimer's disease: the CERAD experience, Part XV. Neurology 46, 1592 (1996).
8. R. J. Bateman et al., Ann Neurol, (Mar 18, 2009).
9. R. J. Bateman et al., Nat Med 12, 856 (Jul, 2006).
10. R. J. Bateman, L. Y. Munsell, X. Chen, D. M. Holtzman, K. E. Yarasheski, J Am Soc Mass Spectrom 18, 997 (Jun, 2007).
11. D. L. Elbert, K. G. Mawuenyega, E. A. Scott, K. R. Wildsmith, R. J. Bateman, J Proteome Res 7, 4546 (Oct 3, 2008).
12. K. E. Yarasheski, K. Smith, M. J. Rennie, D. M. Bier, Biol Mass Spectrom 21, 486 (Oct, 1992).

### Example 4. Aβ42 metabolism is altered in CNS samples

In our latest data analysis of the late-onset Alzheimer's disease Aβ metabolism, we are finding distinct patterns of Aβ42 metabolism compared to the other variants. The relative labeling (H:L ratio of ¹³C₆-Leu/¹²C₆-Leu) was calculated for various Aβ variants in CSF samples taken from a normal individual with no amyloid deposition by PIB (**FIG. 11A**), and an AD patient which has been shown to have amyloid deposition by PIB (**FIG. 11B**). These results show that Aβ42 metabolism is altered in AD individuals. The clearance portion of the Aβ42 curve seems to be consistently altered.

The same analysis was expanded to include data from 50 AD patients. The relative labeling (H:L ratio of ¹³C₆-Leu/¹²C₆-Leu) was calculated for various Aβ42 and Aβ40 variants, and the ratio of Aβ42:Aβ40 relative labeling was calculated in CSF with 95% confidence interval bands grouped by amyloid status (PET PIB scans) for each timepoint in CSF (**FIG. 12**). Note there is highly significant difference by groups in most hours (higher in hours 6 to 15, and lower in hours 24 to 30). In **FIG. 12** and when plotted individually (**FIG. 13**), hours 10 and 27 are significant.

### Example 5. Aβ42 metabolism is altered in blood samples

The same analysis as in Example 5 may be performed using blood samples from the 50 AD patients. The relative labeling (H:L ratio of ¹³C₆-Leu/¹²C₆-Leu) may be calculated for various Aβ42 and Aβ40 variants, and the ratio of Aβ42:Aβ40 relative labeling may be calculated in blood with 95% confidence interval bands grouped by amyloid status (PET PIB scans) for each timepoint in blood. For instance, see **FIG. 14****.**

### Example 6. Additional in vivo data using the SILK method

It was hypothesized that simple measures that summarize some aspect of the SILK tracer curve of amyloid beta (Aβ) may provide diagnostic or prognostic information about patients with AD, at risk of AD, or suspected of having AD. To test the above hypothesis, discrimination between three groups of patients was attempted based on the ratio of the percent of Aβ42 labeling to the percent of Aβ40 percent calculated during the downturn of the Aβ SILK tracer curve. *In vivo* SILK studies were performed in patients with *PSEN1* or *PSEN2* mutations that were PIB positive by PET (MC+), patients with *PSEN1* or *PSEN2* mutations that were PIB negative by PET (MC-), and non-carrier mutation carrier sibling controls (NC) as described elsewhere (Bateman RJ et al 2006 Nature Medicine 12(7):856 and US 7892845) unless otherwise noted. Briefly, subjects were administered isotope-labeled leucine (¹³C₆-leucine) for 9 hours via intravenous infusion. CSF samples (6 mL/sample) were collected 23 hours and 24 hours after the start of the infusion of labeled amino acid. Quantitative measurements of labeled and unlabeled Aβ42 and Aβ40 were obtained by tandem mass spectrometry, and the ratio of labeled:unlabeled Aβ42 and labeled:unlabeled Aβ40 was calculated for each timepoint. These ratios represent the percent labeled of each Aβ isoform at 23 hours and 24 hours post infusion.

A diagnostic threshold of 0.9 was defined in these experiments, such that a ratio of Aβ42 percent labeled / Aβ40 percent labeled below 0.9 classified a subject as AD positive and a ratio of Aβ42 percent labeled / Aβ40 percent labeled above 0.9 classified a subject as AD negative. To determine whether the ratio of Aβ42 percent labeled / Aβ40 percent labeled at 23 hrs post infusion was differentiated between the three groups of patients, the ratio obtained for each patient was graphed versus PIB staining. As can be seen in **FIG. 15A****,** a threshold of 0.9 for this ratio clearly differentiates the majority of MC+ subjects from the NC subjects (6/7 MC+ subjects were below the threshold, while 11/12 NC subjects were above the threshold). Within the MC- group, 3/4 of the subjects were below the threshold. It is possible, however, that subjects in the MC- group were in the early stages of AD. Similarly, the average of the 23 hour and 24 hour labeling percentages may be compared as a ratio between Aβ42 and Aβ40. Aβ42 percent labeled / Aβ40 percent labeled at 23 hrs post infusion and 24 hrs was differentiated between the three groups of patients, the ratio obtained for each patient was graphed versus PIB staining. As can be seen in **FIG. 15B****,** with this measure, 7/7 MC+ subjects are below the threshold, while 11/12 NC are above the threshold. For the MC- group, 2/4 subjects are below the threshold.

These data may be compared to a simple measure that uses the results from the full kinetic model. In this case, the parameter kex42, which describes the rate of entry of Aβ42 into the exchange compartment, is multiplied by 10 and then added to the ratio of the rate constants for irreversible loss for Aβ42 versus Aβ40. As shown in **FIG. 15C****,** a threshold of 1.75 shows that 6/7 of the MC+ subjects are above the threshold, with 12/12 of the NC subjects below the threshold. For the MC- group, 2/4 subjects are below the threshold.

These examples indicate that simple measures that summarize some aspect of the SILK tracer curve may be diagnostic of AD. This also indicates that short term collection of CSF may be sufficient to diagnose changes in Aβ42 kinetics.

## Claims

1. A method of diagnosing Asp amyloidosis in a subject, the method comprising measuring the *in vivo* relative labeling of Aβ42 and at least one other Aβ variant, in a cerebral spinal fluid (CSF) sample or a blood sample obtained from the subject, and calculating the ratio of relative labeling of the Aβ42 variant to the relative labeling of the other Aβ variant, wherein a ratio other than 1 indicates the presence of Aβ amyloidosis.

2. The method of claim 1, wherein the *in vivo* relative labeling of each Aβ variant is measured by:
a. detecting and quantifying by mass spectrometry the amount of the labeled Aβ variant and the amount of the unlabeled Aβ variant in the CSF sample or the blood sample obtained from the subject, wherein the sample was obtained from the subject after administration of a labeled amino acid to the subject; and
b. calculating the ratio of the labeled Aβ variant to the unlabeled Aβ variant, wherein the ratio of labeled Aβ variant to unlabeled Aβ variant represents the relative labeling of said Aβ variant in the subject.

3. The method of claim 2, wherein the sample is a CSF sample.

4. The method of claim 3, wherein the relative labeling of each Aβ variant is measured from a CSF sample obtained 10 hours after administration of the labeled moiety to the subject has begun, and a ratio of the relative labelling of Aβ42 to the other Aβ variant is more than 1, thereby indicating the presence of Aβ amyloidosis.

5. The method of claim 4, wherein the ratio of relative labeling of Aβ42 to the other Aβ variant is 1.1 or greater, thereby indicating the presence of Aβ amyloidosis.

6. The method of claim 3, wherein the relative labeling of each Aβ variant is measured from a CSF sample obtained 27 hours after administration of the labeled moiety to the subject has begun, and a ratio of the relative labeling of Aβ42 to the other Aβ variant is less than 1, thereby indicating the presence of Aβ amyloidosis.

7. The method of claim 2, wherein the sample is a blood sample.

8. The method of claim 2, wherein the label is a non-radioactive isotope selected from the group consisting of ²H, ¹³C, ¹⁵N, ¹⁷O, ¹⁸O, ³³S, ³⁴S, and ³⁶S.

9. The method of claim 8, wherein the non-radioactive isotope is ¹³C attached to or a component of leucine.

10. The method of claim 2, wherein the labeled amino acid is ¹³C₆-leucine or ¹³C₆-phenylalanine.

11. The method of claim 2, wherein a ratio other than 1 during the production phase of the Aß variant or the clearance phase of the Aß variant indicates the presence of Aβ amyloidosis.

12. The method of claim 11, wherein the ratio is 1.04 or greater during the production phase, thereby indicating the presence of Aβ amyloidosis.

13. The method of claim 11, wherein the ratio is 0.9 or less during the clearance phase, thereby indicating the presence of Aβ amyloidosis.

14. The method of claim 3, wherein the relative labeling of each Aβ variant is measured from a CSF sample obtained 6, 7, 8, 9 or 10 hours after administration of the labeled moiety to the subject has begun, and the ratio of the relative labeling of Aβ42 to the other Aβ variant is 1.2 or greater, thereby indicating the presence of Aβ amyloidosis.

15. The method of claim 3, wherein the relative labeling of each Aβ variant is measured from a CSF, sample obtained 23, 24, 25, 26, 27, 28, 29, 30 or 31 hours after administration of the labeled moiety to the subject has begun, and the ratio of the relative labeling of Aβ42 to the other Aβ variant is 0.9 or less, thereby indicating the presence of Aβ amyloidosis.

## Patentansprüche

1. Verfahren zur Diagnose von Aβ-Amyloidose bei einem Individuum, wobei das Verfahren das Messen der relativen Markierung *in vivo* von Aβ42 und mindestens einer weiteren Aß-Variante in einer Rückenmarksflüssigkeits- (CSF) Probe oder einer von dem Individuum erhaltenen Blutprobe sowie Berechnen des Verhältnisses der relativen Markierung der Aß42-Variante zu der relativen Markierung der weiteren Aß-Variante umfasst, wobei ein Verhältnis ungleich 1 das Vorliegen von Aβ-Amyloidose anzeigt.

2. Verfahren nach Anspruch 1, wobei die relative Markierung *in vivo* jeder Aß-Variante gemessen wird durch:
a. Erfassen und Quantifizieren der Menge der markierten Aß-Variante und der Menge der unmarkierten Aß-Variante in der von dem Individuum erhaltenen Rückenmarksflüssigkeitsprobe oder Blutprobe durch Massenspektrometrie, wobei die Probe von dem Individuum nach Verabreichung einer markierten Aminosäure an das Individuum erhalten wurde, und
b. Berechnen des Verhältnisses der markierten Aß-Variante zu der unmarkierten Aβ-Variante, wobei das Verhältnis von markierter Aß-Variante zu unmarkierter Aβ-Variante die relative Markierung der Aß-Variante in dem Individuum darstellt.

3. Verfahren nach Anspruch 2, wobei es sich bei der Probe um eine Rückenmarksflüssigkeitsprobe handelt.

4. Verfahren nach Anspruch 3, wobei die relative Markierung jeder Aß-Variante aus einer Rückenmarksflüssigkeitsprobe gemessen wird, die 10 Stunden nach Beginn der Verabreichung der markierten Einheit an das Individuum erhalten wurde, und ein Verhältnis der relativen Markierung von Aβ42 zu der weiteren Aß-Variante mehr als 1 beträgt, wodurch das Vorliegen von Aβ-Amyloidose angezeigt wird.

5. Verfahren nach Anspruch 4, wobei das Verhältnis der relativen Markierung von Aβ42 zu der weiteren Aß-Variante 1,1 oder mehr beträgt, wodurch das Vorliegen von Aβ-Amyloidose angezeigt wird.

6. Verfahren nach Anspruch 3, wobei die relative Markierung jeder Aβ-Variante aus einer Rückenmarksflüssigkeitsprobe gemessen wird, die 27 Stunden nach Beginn der Verabreichung der markierten Einheit an das Individuum erhalten wurde, und ein Verhältnis der relativen Markierung von Aβ42 zu der weiteren Aß-Variante weniger als 1 beträgt, wodurch das Vorliegen von Aβ-Amyloidose angezeigt wird.

7. Verfahren nach Anspruch 2, wobei es sich bei der Probe um eine Blutprobe handelt.

8. Verfahren nach Anspruch 2, wobei es sich bei der Markierung um ein nichtradioaktives Isotop handelt, das ausgewählt ist aus der Gruppe, bestehend aus ²H, ¹³C, ¹⁵N, ¹⁷O, ¹⁸O, ³³S, ³⁴S und ³⁶S.

9. Verfahren nach Anspruch 8, wobei es sich bei dem nicht-radioaktiven Isotop um ¹³C handelt, das an Leucin gebunden oder ein Bestandteil davon ist.

10. Verfahren nach Anspruch 2, wobei es sich bei der markierten Aminosäure um ¹³C₆-Leucin oder ¹³C₆-Phenylalanin handelt.

11. Verfahren nach Anspruch 2, wobei ein Verhältnis ungleich 1 während der Herstellungsphase der Aß-Variante oder der Ausscheidungsphase der Aß-Variante das Vorliegen von Aβ-Amyloidose anzeigt.

12. Verfahren nach Anspruch 11, wobei das Verhältnis während der Herstellungsphase 1,04 oder mehr beträgt, wodurch das Vorliegen von Aβ-Amyloidose angezeigt wird.

13. Verfahren nach Anspruch 11, wobei das Verhältnis während der Ausscheidungsphase 0,9 oder weniger beträgt, wodurch das Vorliegen von Aβ-Amyloidose angezeigt wird.

14. Verfahren nach Anspruch 3, wobei die relative Markierung jeder Aß-Variante aus einer Rückenmarksflüssigkeitsprobe gemessen wird, die 6, 7, 8, 9 oder 10 Stunden nach Beginn der Verabreichung der markierten Einheit an das Individuum erhalten wurde, und das Verhältnis der relativen Markierung von Aβ42 zu der weiteren Aβ-Variante 1,2 oder mehr beträgt, wodurch das Vorliegen von Aβ-Amyloidose angezeigt wird.

15. Verfahren nach Anspruch 3, wobei die relative Markierung jeder Aβ-Variante aus einer Rückenmarksflüssigkeitsprobe gemessen wird, die 23, 24, 25, 26, 27, 28, 29, 30 oder 31 Stunden nach Beginn der Verabreichung der markierten Einheit an das Individuum erhalten wurde, und das Verhältnis der relativen Markierung von Aβ42 zu der weiteren Aß-Variante 0,9 oder weniger beträgt, wodurch das Vorliegen von Aβ-Amyloidose angezeigt wird.

## Revendications

1. Procédé de diagnostic de l'amylose Aβ chez un sujet, le procédé comprenant la mesure du marquage relatif *in vivo* de l'Aβ42 et d'au moins un autre variant d'Aβ, dans un échantillon de liquide céphalorachidien (LCR) ou un échantillon de sang obtenu du sujet, et le calcul du rapport du marquage relatif du variant Aβ42 sur le marquage relatif de l'autre variant d'Aβ, dans lequel un rapport différent de 1 indique la présence d'une amylose Aβ.

2. Procédé selon la revendication 1, dans lequel le marquage relatif *in vivo* de chaque variant d'Aβ est mesuré par :
a. la détection et la quantification par spectrométrie de masse de la quantité du variant d'Aβ marqué et de la quantité du variant d'Aβ non marqué dans l'échantillon de LCR ou l'échantillon de sang obtenu du sujet, dans lequel l'échantillon a été obtenu du sujet après l'administration de l'acide aminé marqué au sujet ; et
b. le calcul du rapport du variant d'Aβ marqué sur le variant d'Aβ non marqué, dans lequel le rapport du variant d'Aβ marqué sur le variant d'Aβ non marqué représente le marquage relatif dudit variant d'Aβ chez le sujet.

3. Procédé selon la revendication 2, dans lequel l'échantillon est un échantillon de LCR.

4. Procédé selon la revendication 3, dans lequel le marquage relatif de chaque variant d'Aβ est mesuré à partir d'un échantillon de LCR obtenu 10 heures après que l'administration de la fraction marquée au sujet a débuté, et qu'un rapport du marquage relatif de l'Aβ42 sur l'autre variant d'Aβ est supérieur à 1, indiquant de cette façon la présence d'une amylose Aβ.

5. Procédé selon la revendication 4, dans lequel le rapport du marquage relatif de l'Aβ42 sur l'autre variant d'Aβ est de 1,1 ou supérieur, indiquant de cette façon la présence d'une amylose Aβ.

6. Procédé selon la revendication 3, dans lequel le marquage relatif de chaque variant d'Aβ est mesuré à partir d'un échantillon de LCR obtenu 27 heures après que l'administration de la fraction marquée au sujet a débuté, et qu'un rapport du marquage relatif de l'Aβ42 sur l'autre variant d'Aβ est inférieur à 1,
indiquant de cette façon la présence d'une amylose Aβ.

7. Procédé selon la revendication 2, dans lequel l'échantillon est un échantillon de sang.

8. Procédé selon la revendication 2, dans lequel le marqueur est un isotope non radioactif choisi dans le groupe constitué de ²H, ¹³C, ¹⁵N, ¹⁷O, ¹⁸O, ³³S, ³⁴S, et ³⁶S.

9. Procédé selon la revendication 8, dans lequel l'isotope non radioactif est le ¹³C fixé à ou un composant de la leucine.

10. Procédé selon la revendication 2, dans lequel l'acide aminé marqué est la ¹³C₆-leucine ou la ¹³C₆-phénylalanine.

11. Procédé selon la revendication 2, dans lequel un rapport différent de 1 durant la phase de production du variant d'Aβ ou la phase de clairance du variant d'Aβ indique la présence d'une amylose Aβ.

12. Procédé selon la revendication 11, dans lequel le rapport est de 1,04 ou supérieur durant la phase de production, indiquant de cette façon la présence d'une amylose Aβ.

13. Procédé selon la revendication 11, dans lequel le rapport est de 0,9 ou inférieur durant la phase de clairance, indiquant de cette façon la présence d'une amylose Aβ.

14. Procédé selon la revendication 3, dans lequel le marquage relatif de chaque variant d'Aβ est mesuré à partir d'un échantillon de LCR obtenu 6, 7, 8, 9 ou 10 heures après que l'administration de la fraction marquée au sujet a débuté, et que le rapport du marquage relatif de l'Aβ42 sur l'autre variant d'Aβ est de 1,2 ou supérieur, indiquant de cette façon la présence d'une amylose Aβ.

15. Procédé selon la revendication 3, dans lequel le marquage relatif de chaque variant d'Aβ est mesuré à partir d'un échantillon de LCR obtenu 23, 24, 25, 26, 27, 28, 29, 30 ou 31 heures après que l'administration de la fraction marquée au sujet a débuté, et que le rapport du marquage relatif de l'Aβ42 sur l'autre variant d'Aβ est de 0,9 ou inférieur, indiquant de cette façon la présence d'une amylose Aβ.
